(19) Europäisches Patentamt
European Patent Office
Office européen des brevets

(11) Veröffentlichungsnummer: **0 032 591**
**B1**

# (12) EUROPÄISCHE PATENTSCHRIFT

(45) Veröffentlichungstag der Patentschrift: **02.02.83**

(51) Int. Cl.³: **C 07 H 15/22**

(21) Anmeldenummer: **80108216.5**

(22) Anmeldetag: **27.12.80**

(54) **Verfahren zur Herstellung von gereinigten Aminoglycosid-Antibiotika.**

(30) Priorität: **11.01.80 DE 3000841**

(43) Veröffentlichungstag der Anmeldung: **29.07.81 Patentblatt 81/30**

(45) Bekanntmachung des Hinweises auf die Patenterteilung: **02.02.83 Patentblatt 83/5**

(84) Benannte Vertragsstaaten: **DE FR GB IT**

(56) Entgegenhaltungen:

**EP-A-0 000 057**
**EP-A-0 000 889**
**EP-A-0 002 450**
**EP-A-0 007 996**
**EP-A-0 021 215**

(73) Patentinhaber: **BAYER AG, Zentralbereich Patente, Marken und Lizenzen, D-5090 Leverkusen 1, Bayerwerk (DE)**

(72) Erfinder: **Stadler, Peter, Dr., Am Ideck 8, D-5657 Haan (DE)**
Erfinder: **Koebernick, Wolfgang, Dr., Claudiusweg 3, D-5600 Wuppertal 1 (DE)**
Erfinder: **Samaan, Samir, Dr., Claudiusweg 9, D-5600 Wuppertal 1 (DE)**
Erfinder: **Gau, Wolfgang, Dr., Am Eckbusch 39/56, D-5600 Wuppertal 1 (DE)**

EP 0 032 591 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von gereinigten Aminoglycosid-Antibiotika

Die Erfindung betrifft ein verbessertes Verfahren zur Isolierung und Reinigung von Aminoglycosid-Antibiotika der Formel

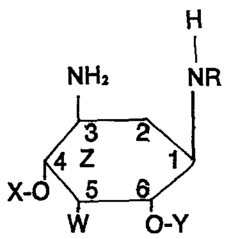

(I)

worin

X einen Rest der Formeln

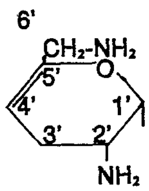

,

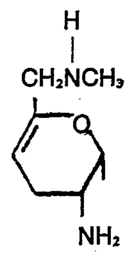

,

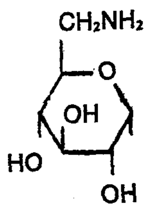

,

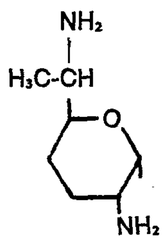

,

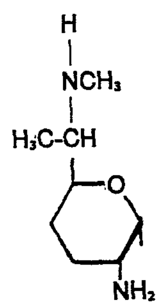

,

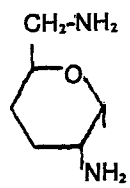

,

Y einen Rest der Formeln

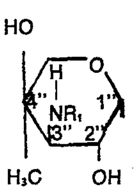

,

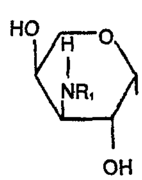

,

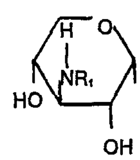

R Wasserstoff oder Ethyl,

$R_1$ eine $C_1$-$C_6$-Alkylgruppe, vorzugsweise Methyl oder Ethyl, ein Rest Z oder W Wasserstoff und der andere Rest Z oder W Wasserstoff oder Hydroxy bedeuten.

Das erfindungsgemässe Verfahren dient insbesondere zur Reinigung bzw. Herstellung von Sisomicin, 5-Episisomicin, Netilmicin, Gentamicin und 3''-N-Desmethyl-3''-N-ethylsisomicin.

Die Verbindungen der Formel (I) sind bekannte Aminoglycosid-Antibiotika. Sie werden zum Teil biosynthetisch durch Kultivierung eines das betreffende Antibiotikum erzeugenden Stammes in einem wässrigen Nährmedium unter bestimmten Bedingungen oder durch chemische Abwandlung eines nach dem vorstehend genannten biosynthetischen Verfahren erhaltenen Antibiotikums erhalten.

Die vorliegende Erfindung betrifft nun ein mehrstufiges Aufarbeitungsverfahren gemäss Patentanspruch 1, das sich einer neuen, vorteilhaften Konzeption zur Isolierung und Reinigung von Aminoglykosiden bedient: Sie kombiniert eine selektive Lipophilisierung der betreffenden Verbindung der Formel I im fermentativ gewonnenen Rohprodukt mit gezielter Flüssig-Flüssig-Extraktion dieses lipophilisierten Produktes.

Die Lipophilisierung wird dadurch erreicht, dass in den Verbindungen der Formel I enthaltene Aminogruppen mit geeigneten Schutzgruppen versehen werden. Nachdem das gewünschte Aminoglykosidderivat durch Flüssig-Flüssig-Extraktion in reiner Form abgetrennt worden ist, werden die Schutzgruppen wieder abgespalten, und man erhält das gewünschte Aminoglykosid der Formel I in reiner Form.

Im Falle der biosynthetischen Verbindungen wird die Fermentationsbrühe vorgereinigt und die Aminogruppen der gewünschten Verbindung sowie der unerwünschten Begleitsubstanzen werden mit für den Einzelfall ausgewählten Schutzgruppenreagenzien umgesetzt.

Im Falle der synthetisch abgewandelten Verbindungen wird ebenfalls von der vorgereinigten Fermentationsbrühe eines biosynthetisch erzeugten Aminoglykosids ausgegangen, woran sich eine selektive Umsetzung mit für den Einzelfall ausgewählten Schutzgruppenreagenzien anschliesst. Mit den so erhaltenen selektiv blokkierten Zwischenprodukten wird die gewünschte chemische Reaktion, beispielsweise die Einführung einer Ethylgruppe in die 1-Position oder der Austausch der Methylgruppe gegen eine Ethylgruppe in der 3''-Position, durchgeführt, woran sich gegebenenfalls eine weitere Reaktion mit Schutzgruppenreagenzien anschliesst.

Es wurde gefunden, dass sich aus den vorgereinigten ganz oder teilweise mit Schutzgruppen versehenen Verbindungen der allgemeinen Formel II,

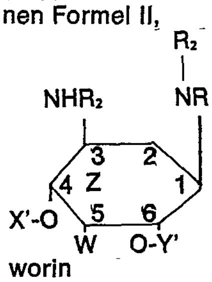

(II)

worin

X' für einen Rest der Formeln

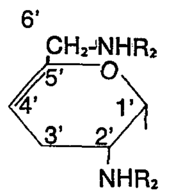 , 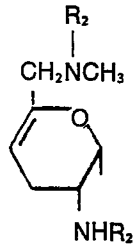 ,

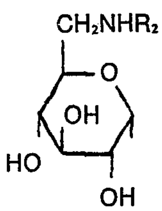 , 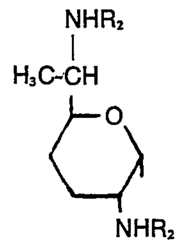 ,

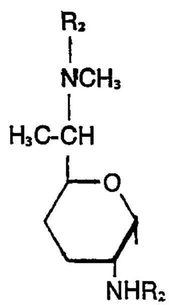 , 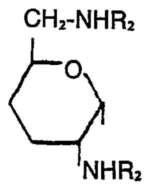 ,

und

Y' für einen Rest der Formeln

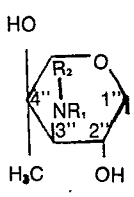 , 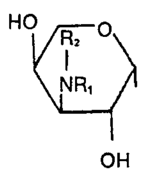 , 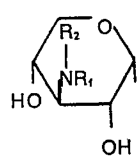

steht, die Reste $R_2$ gleich oder verschieden sind und Wasserstoff oder eine Acyl- oder Arylsulfenyl-Schutzgruppe darstellen, mit der Massgabe, dass maximal zwei der Reste $R_2$ für Wasserstoff stehen, und R, $R_1$, Z und W die oben angegebene Bedeutung haben, die Verbindungen der Formel I in reiner Form, hoher Ausbeute und auf einfache Art erhalten lassen, wenn man die vorgereinigten, mit Schutzgruppen versehenen — und dadurch lipophilisierten — und gegebenenfalls chemisch abgewandelten Substanzen einer Flüssig-Flüssig-Extraktion im Zweiphasensystem aus für den jeweiligen Fall ausgewählten Lösungsmitteln unterwirft, aus den Extrakten die gewünschten, gereinigten, Schutzgruppen enthaltenden Verbindungen abtrennt, gegebenenfalls weitere chemische Abwandlungen durchführt und die Schutzgruppen in an sich bekannter Weise abspaltet.

Die Ermittlung der für den Einzelfall geeigneten Schutzgruppen und des Flüssig-Flüssig-Extraktionssystems kann leicht durch Vorversuche, unterstützt durch geeignete analytische Methoden, vorzugsweise durch Craig-Verteilung oder Hochdruck-Flüssigkeits-Chromatographie (HPLC) erfolgen.

Die bisher verwendeten Isolierungs- und Reinigungsverfahren sind dagegen vielstufig, umständlich und mit hohem Ausbeuteverlusten behaftet. Beispielsweise wird Sisomicin (Antibiotikum 66-40) auf folgende Weise gereinigt (DE-OS 1 932 309, Beispiele 2 bis 5):

Die Fermentationsbrühe wird angesäuert, das Myzel abfiltriert, das Filtrat mit wässrigem Ammoniak neutralisiert und nach Entfernen der Calciumionen mit einem Kationenaustauscherharz behandelt, wodurch das gewünschte Aminoglycosid neben einem beträchtlichen Teil anderer organischer und anorganischer Verbindungen gebunden wird. Das Harz wird mit Wasser gewaschen und durch anschliessendes Eluieren mit wässrigem Ammoniak erhält man Sisomicin als 50%iges Rohprodukt. Dieses Produkt wird durch Ionenaustauscherchromatographie weiter gereinigt, durch Behandeln mit Schwefelsäure in das Sulfat überführt und dieses Sulfat wird durch Säulechromatographie und Umlösen weiter gereinigt und schliesslich in die freie Base zurückgeführt. Die Ausbeute beträgt, bezogen auf das 50%ige Rohprodukt, weniger als 10%.

Die Herstellung der mit Schutzgruppen versehenen Aminoglycosid-Antibiotika (1. Stufe des erfindungsgemässen Verfahrens) ist z. B. aus der EP-OS 57 bekannt, wobei es sich bei den Schutzgruppen um Acyl- oder Arylsulfenylgruppen handelt.

Die Vorreinigung der Fermentationsbrühen geschieht zweckmässigerweise dadurch, dass man sie in an sich bekannter Weise mit einem Kationenaustauscherharz behandelt, an welches das betreffende Aminoglycosid-Antibiotikum gebunden wird, das Harz mit Wasser auswäscht, anschliessend das Aminoglycosid mit wässrigem Ammoniak eluiert und den Ammoniak entfernt.

Geeignete Schutzgruppen sind bekannt, z. B. aus Houben-Weyl, Methoden der organischen Chemie, Band XV, Georg Thieme Verlag, Stuttgart, 1974. Bevorzugte Beispiele solcher Schutzgruppen sind Acylgruppen der Formeln

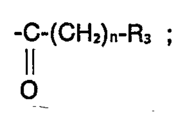 ; 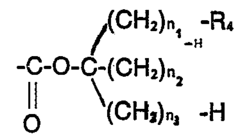

worin

$R_3$ und $R_4$ unabhängig voneinander Wasserstoff oder gegebenenfalls substituiertes Phenyl und

n, $n_1$, $n_2$ und $n_3$ unabhängig voneinander Zahlen von 0 bis 5 bedeuten,

bzw. Sulfenylschutzgruppen der Formel

$-S-R_5$

worin

$R_5$ gegebenenfalls substituiertes Phenyl, Di- oder Triphenylmethyl bedeutet.

Gegebenenfalls substituiertes Phenyl $R_3$, $R_4$ und $R_5$ ist vorzugsweise gegebenenfalls ein- oder zweimal durch Nitro- $C_1$-$C_4$-Alkyl, $C_1$-$C_4$-Alkoxy, Halogen oder Phenyl substituiertes Phenyl.

Zur Herstellung dieser selektiv N-geschützten Aminotrisaccharide setzt man das ungeschützte, biosynthetisch erhaltene Aminotrisaccharid z. B. mit einer Verbindung der folgenden Formeln

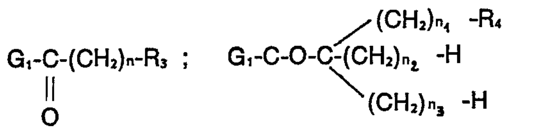

$G_2$-S-$R_5$

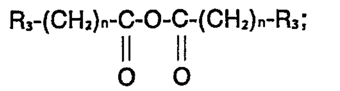

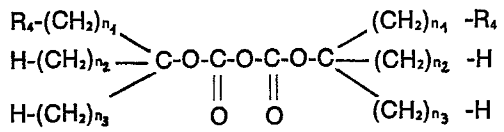

um, wobei

$G_1$ Halogen oder eine andere bei Acylierungsreaktionen gebräuchliche Abgangsgruppe, vorzugsweise eine einen Ester aktivierende Gruppe,

$G_2$ Halogen oder eine andere bei Sulfenylierungsreaktionen gebräuchliche Abgangsgruppe, vorzugsweise eine einen Ester aktivierende Gruppe, bedeuten und

$R_3$, $R_4$, $R_5$, n, $n_1$, $n_2$ und $n_3$ die vorher angegebene Bedeutung besitzen.

Die Einführung der Schutzgruppen wird ausgehend von den fermentativ erhaltenen Rohprodukten in einem Lösungsmittel, bei Temperaturen von etwa −30°C und +50°C, vorzugsweise zwischen etwa 0°C und etwa 25°C, gegebenenfalls in Gegenwart einer Base durchgeführt. Anschliessend wird das Reaktionsprodukt in üblicher Weise aufgearbeitet.

$G_2$ ist vorzugsweise Chlor oder p-Nitrophenyloxy. Als Beispiele für Sulfenylierungsreagentien seien Tritylsulfenylchlorid, o-Nitrophenylsulfenylchlorid, 2,4-Dinitrophenylsulfenylchlorid, 2,4,5-Trichlorphenylsulfenylchlorid, Pentachlorphenylsulfenylchlorid, o-Nitrophenylsulfensäure-p-nitrophenylester, 2,4-Dinitrophenylsulfensäure-p-nitrophenylester, 2,4,5-Trichlorphenylsulfensäure-p-nitrophenylester und Pentachlorphenylsulfensäure-p-nitrophenylester genannt.

Diese reaktiven Sulfensäurederivate sind entweder bereits bekannt (z. B. Houben-Weyl, Methoden der organischen Chemie, Band XV, 1, Seite 203 bis 222, Georg Thieme Verlag, Stuttgart, 1974) oder können nach analogen Verfahren wie die bereits bekannten Verbindungen hergestellt werden.

Als Acylierungsreagentien seien beispielhaft acetanhydrid, Acetylchlorid und Diethylpyrocarbonat genannt, wobei zur Darstellung von N-Alkyl-oxycarbonyl-Derivaten die Verwendung von Dialkylpyrocarbonaten als Schutzgruppenreagentien im allgemeinen besonders zu bevorzugen ist.

Als Verdünnungsmittel für die Umsetzung mit Sulfensäurehalogeniden kommen sowohl inerte organische Lösungsmittel wie Chloroform und Toluol, vorzugsweise aber mit Wasser mischbare Lösungsmittel wie Dioxan, Dimethylformamid und Dimethoxyethan sowie deren Gemische mit Wasser in Frage.

Die Umsetzungen mit aktivierten Estern der oben genannten Sulfensäuren erfolgen vorzugsweise in inerten organischen Lösungsmitteln wie Chloroform, Dimethylformamid, Pyridin oder Gemischen von solchen Lösungsmitteln mit Alkoholen, vorzugsweise Methanol oder Ethanol. Die Darstellung der erfindungsgemässen Acylverbindungen erfolgt in beliebigen inerten organischen Lösungsmitteln, in Wasser oder Gemischen organischer Lösungsmittel mit Wasser, wobei Gemische von Methanol, Ethanol oder Aceton mit Wasser zu bevorzugen sind.

Als Basen können alle in der organischen Chemie üblichen basischen Verbindungen wie Triethylamin, Pyridin, Diazabicyclononen eingesetzt werden; vorzugsweise werden aber Alkalihydroxide bzw. -carbonate wie Natronlauge oder Natriumcarbonat verwendet.

Die Sulfenylierungen bzw. Acylierungen können sowohl bei Normaldruck, aber auch bei erhöhtem Druck durchgeführt werden. Im allgemeinen arbeitet man bei Normaldruck.

Nachstehend seien beispielhaft einige Varianten der 1. Stufe des erfindungsgemässen Verfahrens für verschiedene Aminoglycoside erläutert. Im Falle des Sisomicins kann man beispielsweise so vorgehen, dass eine wässrige Rohsisomicinlösung mit einem Anteil von etwa 10% Sisomicinbase bei 5°C tropfenweise mit Acetanhydrid versetzt wird. Hierbei wird das in der Lösung vorhandene Sisomicin wie auch die übrigen Aminoverbindungen an den primären Aminogruppen acetyliert. Man erhält auf diese Weise ein Gemisch, das 1,3,2',6'-Tetra-N-acetylsisomicin enthält. Unter den gleichen Reaktionsbedingungen reagiert Gentamicin-Rohprodukt zu einem Gemisch, das 1,3,2',6'-Tetra-N-acetylgentamicin C 1a enthält. Verwendet man als Schutzgruppenreagenz Pyrokohlensäuredimethylester oder -diethylester und Rohsisomicin als Substrat, so bildet sich in wässrigem Alkohol bei niedrigen Reaktionstemperaturen 1,3,2',6'-Tetra-N-methoxycarbonylsisomicin bzw. -ethoxycarbonylsisomicin. Andere Verbindungen, die in dem eingesetzten Rohprodukt vorhanden sind und $NH_2$-Gruppen tragen, reagieren an den Aminogruppen ebenfalls mit den Pyrokohlensäureestern unter Bildung der entsprechenden Urethane. Die Umsetzung von Rohsisomicin mit o-Nitrophenylsulfensäure-p-nitrophenylester in einem Gemisch von Methanol und Dichlormethan als Lösungsmittel führt zu 1,3,2',6'-Tetra-N-(o-nitrophenylsulfenyl)-sisomicin. Alle übrigen Substanzen mit primären Aminogruppen, die das Rohprodukt enthält, reagieren ebenfalls mit dem Schutzgruppenreagenz. Die vorstehend aufgeführten Reaktionen verlaufen auch bei den fermentativ gewonnen Rohprodukten glatt und mit hoher Selektivität zu partiell blockierten Derivaten, in denen jeweils die primären Aminogruppen geschützt sind, die sekundären Aminogruppen dagegen noch ein reaktives Wasserstoffatom tragen.

Gewünschtenfalls können auch alle, d.h. primäre und sekundäre Aminogruppen mit Schutzgruppen versehen werden; wobei als Schutzgruppenreagenzien wiederum bevorzugt die auf Seite 6 gnannten Verbindungen verwendet werden. Diese Umsetzung erfolgt in einem inerten Lösungsmittel oder in Lösungsmittelgemischen, gegebenenfalls unter Zusatz von Wasser bei Temperaturen zwischen 0 und 100°C, vorzugsweise zwischen 25 und 70°C, gegebenenfalls in Gegenwart einer Base. Da die sekundären Aminogruppen im allgemeinen weniger reaktiv sind als die primären Aminogruppen, arbeitet man zweckmässigerweise mit einem Überschuss an Schutzgruppenreagenz, hält den Wassergehalt des verwendeten Lösungsmittelgemisches möglichst niedrig und arbeitet evtl. bei erhöhter Temperatur. Als Lösungsmittel für die betreffenden Umsetzungen kommen grundsätzlich alle Solventien in Frage, die die Ausgangsprodukte lösen und selbst nicht oder schlechter mit den betreffenden Schutzgruppenreagenzien reagieren als mit den umzusetzenden Aminogruppen. Beispielsweise seien Chloroform, Methylenchlorid, Ethanol, Methanol, Aceton und Dioxan genannt. Als Hilfsbasen kommen die bereits erwähnten Verbindungen in Frage. Setzt man beispielsweise das bereits genannte 1,3,2',6'-Tetra-N-acetylsisomicin in Ethanol/Wasser (9:1) in Gegenwart von Natriumcarbonat mit Chlorameisensäureisopropylester bei etwa 60°C um, so erhält man 1,3,2',6'-Tetra-N-acetyl-3''-N-isopropyloxycarbonylsisomicin. Die Darstellung der per-N-geschützten Aminotrisaccharide kann auch in einer Stufe erfolgen. So reagiert Sisomicin beispielsweise in Methanol mit überschüssigem Dimethylpyrocarbonat vollständig zu penta-N-Methyloxycarbonylsisomicin.

In vielen Fällen ist es jedoch zweckmässig, penta-N-geschützte Verbindungen mit unterschiedlichen Schutzgruppen zu versehen. Durch geeignete Auswahl der Schutzgruppen lässt sich die Lipophilie bzw. die Hydrophilie der Verbindungen der Formel (II) gezielt so steuern, dass eine optimale Abtrennbarkeit von unerwünschten Nebenprodukten durch Flüssig-Flüssig-Extraktion erreicht wird.

Bei den erfindungsgemäss verwendeten Extraktionssystemen handelt es sich um wässrigorganische Lösungsmittelsysteme. Man arbeitet dabei im allgemeinen so, dass sich das gewünschte Aminotrisaccharidderivat nach der Extraktion in der organischen Phase befindet. Neben Wasser eignen sich als wässrige Phase vor allem wässriges Ammoniumhydroxid, beispielsweise eine gesättigte Lösung von Ammoniak in Wasser oder auch Lösungen mit geringerem prozentualem Gehalt an Ammoniumhydroxid. Anstelle von Ammoniumhydroxid können der Wasserphase auch andere basische Hilfsstoffe zugesetzt werden, wie organische Amine, beispielsweise Methylamin, Dimethylamin und Trimethylamin. Als basische Hilfsstoffe können jedoch auch anorganische Verbindungen wie Alkalihydroxide Verwendung finden.

Als organische Phasen sind mit Wasser nicht oder nur gering mischbare, gegenüber den in die Extraktion eingesetzten Produktgemischen inerte Lösungsmittel geeignet, z. B. Kohlenwasserstoffe wie Toluol, Xylol, Ethylbenzol und Hexan, chlorierte Kohlenwasserstoffe wie Methylenchlorid, 1,2-Dichlorethan und 1,2-Dichlorpropan, Ester wie Essigsäureethylester, Propionsäureethylester, Phosphorsäure-tributylester, Ether wie Diethylether oder Alkohole wie n-Butanol, Propanol-2 und n-Pentanol. Besonders zu bevorzugen sind als organische Phasen auch Gemische von mit Wasser nicht mischbaren Lösungsmitteln mit anderen organischen Lösungsmitteln, beispielsweise Methylenchlorid/Propanol-2, Methylenchlorid/n-Hexan, Methylenchlorid/n-Butanol, 1,2-Dichlorethan/n-Hexan, Butanol/n-Hexan, Methylenchlorid/Propanol-2/n-Hexan und Essigsäureethylester/n-Hexan. Im allgemeinen ist es vorteilhaft, die organischen Phasen, die im Rahmen des Extraktionsverfahrens Verwendung finden, mit Ammoniumhydroxid zu sättigen.

Besonders vorteilhaft können auch solche Zweiphasensysteme für die Extraktion verwendet werden, die sich bilden, wenn man wässrige Amoniumhydroxidlösungen mit einem organischen, nicht oder nur begrenzt mit wässrigem Ammoniumhydroxid mischbaren Lösungsmittel (A) und einem weiteren organischen Lösungsmittel (B) zusammenbringt, wobei (B) wahlweise mit wässrigem Ammoniumhydroxid mischbar sein kann oder nicht mischbar sein kann, anschliessend bis zur Gleichgewichtseinstellung durchmischt, dann die Phasen voneinander trennt und für das betreffende Extraktionsverfahren einsetzt, beispielsweise Zweiphasensysteme, die man aus Mischungen von n-Butanol, Ammoniumhydroxid und n-Hexan oder Methylenchlorid, Ammoniumhydroxid und Propanol-2 erhält.

Das Mengenverhältnis von organischer Phase zu wässriger Phase kann in weiten Grenzen variiert werden. Bevorzugt beträgt dieses Verhältnis 0,5:1 bis 30:1, ganz besonders bevorzugt 5:1 bis 20:1. Im Extrakt kann der Gehalt an der gewünschten Aminotrisaccharidverbindung durch die Menge des Extraktionsmittels und die Zahl der Extraktionsstufen in weiten Grenzen variiert werden. Man wählt die eingesetzten Extraktionsmittel im allgemeinen so, dass man Extrakte erhält, die das gewünschte Aminotrisaccharidrivat in einer Konzentration von 0,1 bis 15% enthalten.

Bei den zur Extraktion eingesetzten wässrigen Rohlösungen wählt man die Lösungsmittelmenge so, dass sich ein Gehalt an Aminotrisaccharidderivat von zwischen 1 und 20% ergibt, wobei man bevorzugt 2- bis 10%ige Lösungen einsetzt. Die Prozentangaben sind in diesem Fall Gewichtsprozente.

Die für eine optimale Extraktion notwendige Zahl der Extraktionsstufen wird in bekannter Weise durch Vorversuche, z. B. über eine Verteilungskurve, ermittelt.

Die Temperatur der Extraktion kann in weiten Grenzen variiert werden. Im allgemeinen wird bei Temperaturen zwischen 10 und 60°C gearbeitet. Es kann bei Normaldruck, vermindertem Druck oder erhöhtem Druck gearbeitet werden. Als Extraktionseinheiten kommen die bekannten Extraktionssysteme in Frage, z. B. Mischer-Scheider, Zentrifugalextraktoren oder Kolonnensysteme wie Sprühkolonnen, gerührte Kolonnen wie Scheibel-Kolonnen oder pulsierte Kolonnen wie pulsierte Siebbodenkolonnen. In besonders einfachen Fällen können Extraktionen auch durch Mischen und Abscheiden in einem Reaktionsgefäss, z. B. in einem Rührwerkskessel, durchgeführt werden. Im Labormassstab können in bekannter Weise auch Schiedetrichter zur Durchführung der Extraktion eingesetzt werden. Bei der Verwendung von Kolonnen wird die Extraktion vorzugsweise als Gegenstromextraktion durchgeführt.

Diejenige Extraktions-Phase, die das gewünschte Aminotrisaccharidderivat enthält, also im allgemeinen die organische Phase, wird zur Isolierung des gewünschten Produktes aufkonzentriert oder vollständig eingedampft, wobei man zur Schonung des Produktes vorzugsweise solche Verdampfereinheiten wählt, in denen die Verweilzeit des Produktes möglichst gering ist, beispielsweise Fallstrom- oder Dünnschichtverdampfer. Die Verdampfung des Lösungsmittels wird vorzugsweise bei vermindertem Druck durchgeführt.

Mit dem erfindungsgemässen Verfahren können nicht nur Aminoglykosidantibiotika von ihren Verunreinigungen getrennt werden, es ist auch möglich, Aminoglycosidgemische, wie sie durch Kultivierung der entsprechenden Mikroorganismen entstehen, in ihre Komponenten aufzutrennen.

Gentamicin besteht aus 3 eng verwandten Komponenten der Formel

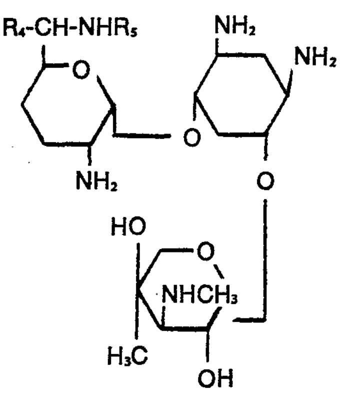

Gentamicin $C_1$ $R_4 = R_5 = CH_3$
Gentamicin $C_2$ $R_4 = CH_3$; $R_5 = H$
Gentamicin $C1_a$ $R_4 = R_5 = H$

Gentamicin enthält etwa 33% $C_1$, 41% $C_2$ und 25% $C_{1a}$. Zur Auftrennung wird Gentamicin unter Kühlen in Wasser mit Acetanhydrid behandelt, wobei alle primären Aminogruppen acetyliert werden. Nun werden nach Aufarbeitung die sekundären Aminogruppen mit Chlorameisensäure-n-butylester in wässrigem Ethanol in die entsprechenden Urethangruppierungen überführt. Man erhält so ein Gemisch von Verbindungen, das im Labormassstab durch stufenweise flüssig-flüssig-Extraktion in einem Schütteltrichter aufgetrennt wird. Als wässrige Phase wird dabei konzentriertes, wässriges Ammoniumhydroxid verwendet, und als organische Phase benutzt man die Unterphase des Zweiphasensystems, welches sich beim Mischen von 7 Vol.-Teilen Methylenchlorid, 0,4 Vol.-Teilen Propanol-2 und 1 Vol.-Teil konzentriertem Ammoniumhydroxid bildet. Hierbei geht das Gentamicin $C_1$-Derivat in die organische Phase, die beiden übrigen Gentamicin-Derivate verbleiben in der wässrigen Phase. Die organische Phase kann gegebenenfalls noch mit wässrigem Ammoniak gegengewaschen werden.

Im erfindungsgemässen Verfahren können die Verbindungen der allgemeinen Formel (II), im Falle dass mindestens einer der Reste $R_2$ für Wasserstoff steht, vor und/oder nach der Extraktionsstufe einer chemischen Derivatisierung an der oder den ungeschützten Aminogruppe(n) unterzogen werden. Hierfür kommen u. a. Acylierungen oder reduktive Alkylierung der Aminogruppe mit Aldehyden in Betracht, wie sie z. B. in den DE-OSen 27 12 160, 27 26 712, 27 53 769, 28 32 268, 29 21 973 und 29 24 659 beschrieben werden.

Im erfindungsgemässen Verfahren können die Verbindungen der allgemeinen Formel (II), im Falle dass mindestens einer der Reste $R_2$ für Wasserstoff steht, vor und/oder nach der Extraktionsstufe einer chemischen Derivatisierung an der oder den ungeschützten Aminogruppe(n) unterzogen werden. Hierfür kommen u. a. Acylierungen oder reduktive Alkylierung der Aminogruppe mit Aldehyden in Betracht, wie sie z. B. in den DE-OSen 27 12 160, 27 26 712, 27 53 769, 28 32 268, 29 21 973 und 29 24 659 beschrieben werden.

Eine besonders interessante Verfahrensvariante besteht darin, ein rohes, fermentativ gewonnenes Aminotrisaccharid der Formel (I) in das entsprechende 1,3,2',6'-Tetra-N-acyl- oder -sulfenyl-Derivat der Formel (II) überzuführen, die 3"-Methylaminogruppe zu alkylieren, vorzugsweise zu ethylieren, gegebenenfalls dieses Derivat durch Gegenstromextraktion rein zu isolieren, anschliessend die erhaltene tertiäre 3"-Aminogruppe zu demethylieren, danach gegebenenfalls die 3"-Alkylaminogruppe mit einer Schutzgruppe zu versehen und der erfindungsgemässen Extraktion zu unterwerfen.

Im folgenden soll die erfindungsgemässe Verfahrensweise am Beispiel der Herstellung von 1,3,2',6'-Tetra-N-acetyl-3"-N-desmethyl-3"-N--ethyl-3"-N-isopropyloxycarbonyl-sisomicin beschrieben werden.

Rohsisomicin, welches wie üblich durch Fermentation, Behandlung mit Kationenaustauscher und Eluieren des Harzes mit wässrigem Ammoniak erhalten wurde, wird wie vorstehend beschrieben mit Acetanhydrid in Wasser zum 1,3,-2',6'-Tetra-N-acetylsisomicin umgesetzt. Anschliessend erfolgt eine reduktive Alkylierung der 3"-Methylaminogruppe mit Acetaldehyd und Natriumboranat zum 3"-N-Ethylderivat.

Dieses tertiäre Amin wird nun mit einem Oxidationsmittel, z. B. Kaliumhexacyanoferrat-(III) in wässrigem Methanol, in Anwesenheit einer Base, z. B. Natriumhydroxid, oxidativ demethyliert unter Bildung der sekundären 3"-Ethylaminoverbindung. Die anschliessende Umsetzung mit Chlorameisensäureisopropylester in wässrigem Alkohol (bei ca. 60°C) unter Anwesenheit von Natriumcarbonat als Base liefert dann ein rohes Penta-N-acylprodukt, welches zur erfindungsgemässen Reinigung einer Flüssig-Flüssig-Extraktion unterworfen wird.

In einer anderen Ausführungsform des Verfahrens kann eine Reinigung des wie vorstehend berschrieben erhaltenen tertiären Amins durch kontinuierliche Gegenstromextraktion erfolgen. Das dann erhaltene reine 1,2',3,6'-Tetra-N-acetyl-3"-N-ethyl-sisomicin kann dann wie beschrieben oxidativdealkyliert werden.

Eines der bevorzugten Verfahren zur Herstellung der rohen 3"-N-alkylierten Derivate der 4,6-Di-O-(aminoglykosyl)-1,3-diaminocyclitole der Formel (II), die Aminoschutzgruppen in allen Stellungen ausser in Stellung 3" enthalten, besteht darin, dass man die entsprechenden tetra-N-geschützten Verbindungen oder deren Säureadditionssalze mit einem Aldehyd in Gegenwart eines Wasserstoff-Donor-Reduktionsmittels umsetzt und den Ansatz in an sich bekannter Weise aufarbeitet.

Dieses Verfahren, bei dem die 3"-Aminogruppe in einem 4,6-Di-O-(aminoglycosyl)-1,3-diaminocyclitol mit einem Aldehyd reagiert und gleichzeitig in situ reduziert wird, wird gewöhnlich bei Raumtemperatur in Gegenwart von Luft durchgeführt, obwohl es günstiger sein kann, die Reaktion unter Inertgas (Argon, Stickstoff) durchzuführen. Die Reaktion ist gewöhnlich sehr rasch, oft in weniger als 60 Minuten, vollendet, was durch dünnschichtchromatographische Bestimmungen festgestellt werden. kann.

Wasserstoff-Donor-Reduktionsmittel, die bei diesem Verfahren Verwendung finden, umfassen Dialkylaminoborane (z. B. Dimethylaminoboran, Diethylaminoboran und vorzugsweise Morpholinoboran), Tetraalkylammoniumcyanoborhydride (z. B. Tetrabutylammoniumcyanoborhydrid), Alkalimetallborhydride (z. B. Natriumborhydrid) und Alkalimetallcyanoborhydrid (z. B. Lithiumcyanoborhydrid und Natriumcyanoborhydrid).

Das Verfahren wird üblicherweise in einem inerten Lösungsmittel durchgeführt. Das Lösungsmittel kann ein organisches oder anorganisches sein, in dem das selektiv geschützte 4,6-Di-O-(aminoglycosyl)-1,3-diaminocyclitol und die anderen Reagentien löslich sind und das unter den Reaktionsbedingungen nach Möglichkeit Nebenreaktionen herabsetzt oder verhindert. Obwohl wasserfreie aprotische Lösungsmittel mit Vorteil eingesetzt werden können (z. B. Tetrahydrofuran, wenn das Reduktionsmittel Morpholinoboran ist), wird gewöhnlich doch ein protisches Lösungsmittel verwendet. Als solches eignet sich z. B. ein niederes Alkanol oder Wasser oder ein wässriges niedriges Alkanol oder andere Lösungsmittelsysteme, die Wasser enthalten wie z. B. wässriges Dimethylformamid, wässriges Hexamethylphosphoramid, wässriges Tetrahydrofuran oder wässriger Ethylenglycoldimethylether.

Das Verfahren wird gewöhnlich in einem pH-Bereich von 1 bis 11 und vorzugsweise bei pH 4 bis 8 durchgeführt.

Ein anderes erfindungsgemässes Verfahren zur Herstellung der 3"-N-alkylierten Aminotrisaccharidderivate besteht in der Alkylierung der betreffenden tetra-N-geschützten Aminotrisaccharide mit unblockierter 3"-Methylaminogruppe mit Alkylhalogeniden

$R_1$-Hal

in denen $R_1$ die oben angegebene Bedeutung hat und Hal Halogen, wie Chlor, Brom oder Jod bedeutet.

Solche Alkylierungen von Aminen sind bereits literaturbekannt (s. z. B. Houben-Weyl, Methoden der organischen Chemie, Bd. XI, 1, Georg Thieme Verlag, Stuttgart 1957). Man arbeitet dabei vorzugsweise in Gegenwart eines unter den Reaktionsbedingungen inerten Verdünnungsmittels, bevorzugt eines solchen, in dem sich die Reaktionspartner gut lösen. Bevorzugte Verdünnungsmittel dieser Art sind Ether wie Tetrahydrofuran, Ethylenglykoldimethylether oder Dioxan, Ketone wie Aceton oder Methylethylketon, Alkohole, Dimethylacetamid und Dimethylformamid. Die Verwendung von Dimethylformamid als Lösungsmittel ist hierbei besonders zu bevorzugen. Je nach Reaktivität des eingesetzten Alkylhalogenids verwendet man 1-10 Moläquivalente Alkylierungsmittel und arbeitet bei pH-Werten von etwa 5 bis 12. Zu bevorzugen ist dabei die Verwendung einer Hilfsbase zum Abfangen des bei der Reaktion freiwerdenden Halogenwasserstoffs. Beispiele für entsprechende Basen sind Alkalimetallhydroxide, Erdalkalimetallhydroxide, Alkalimetall- und Erdalkalimetallcarbonate, Erdalkalimetalloxide, Carbonate und Oxide von Schwermetallen wie z. B. Bleicarbonat und Silbercarbonat sowie Quecksilberoxid oder Silberoxid. Es können prinzipiell alle unter den Reaktionsbedingungen stabilen Verbindungen, die in der Lage sind den gebildeten Halogenwasserstoff abzufangen, als Hilfsbasen verwendet werden.

Es kann vorteilhaft sein, dass man die als Rohprodukte anfallenden tetra-N-geschützten Aminotrisaccharide mit tertiärer 3"-Aminogruppe zur Reinigung einer Flüssig-Flüssig-Extraktion unterwirft und das so gereinigte Produkt dann wie nachstehend beschrieben demethyliert.

Eines der bevorzugten Verfahren zur Abspaltung der 3"-N-Methylgruppe aus den wie vorstehend beschrieben erhaltenen rohen oder extraktiv gereinigten Trisacchariden mit tertiärer 3"-Aminogruppe ist die oxidative Demethylierung mit üblichen Oxidationsmitteln.

Beispiele für Oxidationsmittel sind Schwermetallsalze, Peroxide, Halogene, Halogensauerstoffsäuren und deren Salze, Stickstoffoxide sowie molekularer Sauerstoff. Bevorzugte Oxidationsmittel sind Permanganate, Manganate, Mangandioxid, Chromtrioxid, Bichromate, Chromate, Alkylchromate, Chromylchlorid, Selendioxid, Kobalt(III)-Salze, Cer(IV)-Salze, Kaliumhexacyanoferrat(III), Kupferoxid, Bleioxid, Quecksilberoxid, Gemische von Wasserstoffperoxid mit Eisen(III)-Salzen, Eisen(II)-Salze, Selenoxid, Osmiumtetroxid, Vanadate, Wolframsäure und/oder Chromsäure, Bleitetraacetat, Chlor, Brom, Jod, Hypochlorate, Chlorite, Hypobromate, Bromate, Perjodate, Distickstoffmonoxid, Stickstoffdioxid und Luft. Bei Verwendung von molekularem Sauerstoff werden vorzugsweise Edelmetalle wie Platin, Palladium, Rhodium, Ruthenium oder Rhenium sowie Nickel als Katalysatoren verwendet.

Besonders bevorzugte Oxidationsmittel sind Mangandioxid, Kaliumhexacyanoferrat (III) und Kaliumpermanganat.

Die Spaltungsreaktion wird vorzugsweise in Gegenwart eines unter den Reaktionsbedingungen inerten Verdünnungsmittels, bevorzugt eines solchen, in dem sich die Reaktionsteilnehmer lösen, durchgeführt. Geeignete Verdünnungsmittel der genannten Art sind Wasser oder Gemische von Wasser mit Methanol, Ethanol, i-Propanol, Tetrahydrofuran, Dimethylformamid, Dioxan, Pyridin, Ethylenglykoldimethylether und Aceton.

Die Umsetzung wird je nach Art des verwendeten Oxidationsmittels bei einem pH-Wert von 3 bis 12 durchgeführt. Die Einstellung des pH-Wertes kann durch Zusatz einer entsprechenden Säure oder Base erreicht werden. Dabei sind solche Säuren oder Basen zu verwenden, die die Ausgangsverbindungen oder die Endprodukte nicht zersetzen und keine Aktivitätsverringerung der Oxidationsmittel hervorrufen. Vielmehr ist es wünschenswert, dass sie die Aktivität der Oxidationsmittel erhöhen. Als anorganische Säuren können beispielsweise Salzsäure oder Schwefelsäure und als organische Säuren beispielsweise Essigsäure oder Ameisensäure verwendet werden. Beispiele für entsprechende Basen sind Ammoniumhydroxid, Alkalimetallhydroxide, Erdalkalimetallhydroxide, Alkalimetallcarbonate, Erdalkalimetallcarbonate, Alkalimetallalkoholate und Alkali- und Erdalkalimetallsalze von Carbonsäuren.

Die Einstellung des pH-Wertes kann entweder vor Beginn der Reaktion oder während der Reaktion vorgenommen werden.

Die Umsetzung wird bei Temperaturen von etwa −30°C bis etwa 100°C, vorzugsweise von etwa −20° bis etwa 0°C durchgeführt. Die Reaktionsdauer beträgt eine halbe Stunde bis 50 Stunden. Im allgemeinen wird die Umsetzung bei Normaldruck ausgeführt.

Die Abspaltung der 3"-N-Methylgruppe gelingt auch durch Umsetzung mit Chlorameisensäureestern in Gegenwart von Hilfsbasen, wobei die Methylgruppe durch die entsprechende Alkyl- oder Aryloxycarbonylgruppe ersetzt wird und Penta-N-blockierte Verbindungen gebildet werden.

Im letzten Teil des erfindungsgemässen Verfahrens werden die Schutzgruppen von den Aminogruppen in üblicher Weise abgespalten.

Die Sulfenylgruppen können sowohl mit Nukleophilen wie Schwefelwasserstoff, Thiophenol oder 2-Mercaptobenzthiazol, gegebenenfalls auch in Gegenwart von Mineralsäuren oder auch durch Erhitzen mit anorganischen Basen wie Alkali- oder Erdalkalihydroxiden, evtl. auch unter erhöhtem Druck, abgespalten werden.

Acylschutzgruppen wie Acetyl, Ethyloxycarbonyl oder t-Butyloxycarbonyl können mit wässrigem Alkali- oder Erdalkalihydroxid oder mit Säuren wie Trifluoressigsäure oder Bortrifluoridetherat in organischen Lösungsmitteln bzw. Mischungen von organischen Lösungsmitteln in Wasser abgespalten werden.

Die nach Abspaltung der Schutzgruppen erhaltenen Endprodukte werden als freie Basen oder in Form pharmazeutisch anwendbarer Säureadditionssalze isoliert.

Das erfindungsgemässe Verfahren stellt gegenüber den bisher bekannten Verfahren eine wesentliche Verbesserung dar. Das erfindungsgemässe Verfahren ist breit einsetzbar und liefert mit verhältnismässig geringem verfahrenstechnischen Aufwand reine Produkte in hohen Ausbeuten.

Die Reinheit der erhaltenen Zwischen- und Endprodukte wurde über Craig-Verteilung densitometrisch oder durch Hochdruck-Flüssigkeitschromatographie (HPLC) bestimmt. Folgende Lösungsmittelsysteme wurden in den Beispielen bei der Dünnschichtchromatographie verwendet:

Laufmittelgemisch G

1000 ml Methanol, 1000 ml Methylenchlorid und 1000 ml 15%iges Ammoniumhydroxid werden 5 Minuten durchgeschüttelt. Die Unterphase wird nach der Abtrennung mit 1% Methanol (= 10 ml) versetzt und zur Chromatographie benutzt.

Laufmittelgemisch E

Mischung aus 200 ml Methylenchlorid, 400 ml Methanol und 100 ml 20%igem Ammoniumhydroxid.

Die in den folgenden Beispielen verwendeten Rohprodukte können so gewonnen werden, dass das entsprechende Fermentationsmedium angesäuert und dann filtriert wird; anschliessend

werden gegebenenfalls Calciumionen mit Oxalsäure entfernt. Die Lösung wird mit wässrigem Ammoniumhydroxid neutralisiert und mit einem Kationenaustauscherharz ($NH_4^{\oplus}$-Form) behandelt, an welches die Aminoglykoside gebunden werden; der Austauscher wird gewaschen und mit wässrigem Ammoniumhydroxid eluiert. Die so erhaltenen Aminotrisaccharid-Rohlösungen werden bis zum Entfernen des Ammoniaks eingedampft.

Beispiel 1
Penta-N-(o-nitrophenylsulfenyl)-sisomicin--Rohprodukt

Zu 442 g 50%igem Rohsisomicin in 400 ml Wasser und 800 ml Tetrahydrofuran werden bei pH 12 bis 14 285 g o-Nitrophenylsulfenylchlorid in 686 ml Tetrahydrofuran sowie 186 ml 8 n Natronlauge gleichzeitig während 30 Minuten unter Rühren getropft. Die Temperatur steigt dabei auf ca. 45°C. Der Rührer wird abgestellt, die Wasserphase abgetrennt und die organische Phase am Rotationsverdampfer im Vakuum eingedampft. Der Rückstand wird im Vakuum getrocknet. Ausbeute an Rohprodukt: 670 g. Im Dünnschichtchromatogramm (Laufmittelsystem Methylenchlorid/Methanol = 85/5) ist die Titelverbindung als Hauptprodukt zu erkennen.
Rf = 0,87.

Beispiel 2
1,2',3,6'-Tetra-N-(o-nitrophenylsulfenyl)-sisomicin

90 g 50%iges Rohsisomicin in 100 ml Methanol und 900 ml Dichlormethan werden mit 130 g o-Nitrophenylsulfensäure-p-nitrophenylester versetzt, das Reaktionsgemisch nach ca. einer Stunde auf 3000 ml Methanol gegossen, der Niederschlag mit 500 ml Dichlormethan digeriert und der Rückstand nach Filtrieren getrocknet. Ausbeute an Rohprodukt 101 g. Im Dünnschichtchromatogramm (Laufmittelsystem Methylenchlorid/Methanol = 9/1) ist die Titelverbindung als Hauptprodukt zu erkennen.
Rf = 0,14.

Beispiel 3
Reinsisomicin aus Rohsisomicin über 1,2',3,6'--Tetra-N-acetyl-3"-N-(butyloxycarbonyl)-sisomicin

3.1 1,2',3,6'-Tetra-N-acetylsisomicin (Rohprodukt)

1020 ml wässrige Rohsisomicinlösung mit einem Sisomicingehalt von 122 g werden mit 400 ml Wasser verdünnt und nach Kühlen auf ca. 5°C langsam mit 240 ml Acetanhydrid versetzt. Anschliessend wird im Vakuum eingedampft und die Essigsäure dabei weitgehend abdestilliert. Restliches Acetat entfernt man durch Behandeln mit Anionenaustauscherharz (Lewatit MP 500, $OH^{\ominus}$-Form) in wässriger Lösung. Nach Abtrennen des Harzes wird im Vakuum zum Sirup eingedampft. Das so erhaltene Rohprodukt enthält 1,2',3,6'-Tetra-N-acetylsisomicin als Hauptprodukt (Rf = 0,21 Laufmittelsystem G).

3.2 1,2',3,6'-Tetra-N-acetyl-3"-N-(n-butyloxycarbonyl)-sisomicin (Rohprodukt)

Das bei 3.1 erhaltene Rohprodukt wird in 840 ml Ethanol und 160 ml Wasser gelöst, dann werden 185 g Natriumcarbonat zugefügt. Bei 50°C tropft man eine Lösung von 185 ml Chlorameisensäure-n-butylester in 185 ml Aceton zu, versetzt dann mit 1500 ml Ethanol, lässt abkühlen und filtriert von den anorganischen Salzen ab. Man dampft im Vakuum zum Sirup ein und erhält die Titelverbindung als Rohprodukt in Form eines braungefärbten Sirups.

3.3 Extraktive Reinigung und Abspaltung der Schutzgruppen, Isolierung von Reinsisomicin

Der bei 3.2 erhaltene Sirup wird in 750 ml konzentriertem wässrigem Ammoniak gelöst. Lipophile Verunreinigungen werden aus dieser Lösung mit zweimal je 350 ml Methylenchlorid extrahiert. Anschliessend gewinnt man das Sisomicinderivat dadurch, dass man die Ammoniaklösung zwanzigmal mit je 1500 ml der Unterphase des Systems Methylenchlorid (7 Vol.-Teile), Propanol-2 (0,8 Vol.-Teile) und konzentriertem wässrigem Ammoniak (1,0 Vol.-Teile) extrahiert. Nach Verdampfen des Extraktionsmittels erhält man 1,2',3,6'-Tetra-N-acetyl-3"-N--(n-butyloxycarbonyl)-sisomicin als farblosen Rückstand, der zur weiteren Reinigung nochmals der oben beschriebenen Extraktion unterworfen wird.

Zur Abspaltung der Schutzgruppen wird das Reinprodukt in 1500 ml Wasser mit 880 g $Ba(OH)_2 \times 8H_2O$ am Rückfluss erhitzt. Nach etwa 8 Stunden werden die Bariumsalze durch Fällen mit $CO_2$ und Filtrieren abgetrennt. Das Filtrat wird mit basischem Ionenaustauscherharz (Lewatit MP 500, $OH^{\ominus}$-Form) entionisiert. Aus der Lösung wird die Sisomicinbase durch Adsorption an Katrionenaustauscherharz (Lewatit CNP-LF, $NH_4^{\oplus}$-Form) entfernt. Das so erhaltene Harzaddukt wird mit Wasser gewaschen. Dann wird die Sisomicinbase mit 5%igem wässrigem Ammoniak eluiert. Das Eluat wird im Vakuum eingedampft, der Rückstand (Sisomicinbase) durch Behandeln mit Schwefelsäure in Wasser in das Sulfat überführt. Nach Gefriertrocknen erhält man Sisomicinsulfat als farblosen Feststoff.

Ausbeute: 151 g ≙ 80% der Theorie;
$[\alpha]_D^{20} = +105°$ (c = 1.0 $H_2O$);
Reinheitsgrad: 96%.

Beispiel 4
Reinsisomicin aus Rohsisomicin über 1,2',3,6'--Tetra-N-acetyl-3"-N-(n-octyloxycarbonyl)-sisomicin

4.1 1,2',3,6'-Tetra-N-acetyl-3''-N-(n-octyl-oxycarbonyl)-sisomicin, Rohprodukt

12,8 g Rohsisomicin (Gehalt an Sisomicin ca. 55%) in 70 ml Wasser werden unter Rühren tropfenweise mit 12 ml Acetanhydrid versetzt. Man dampft im Vakuum ein und löst den Rückstand in 150 ml Wasser. Dann wird durch Rühren mit 150 ml Anionenaustauscherharz (Lewatit MP 500, OH⊖-Form) entionisiert, vom Harz abfiltriert und das Filtrat im Vakuum zum Sirup eingedampft. Diesen löst man in 50 ml Propanol-1, fügt 11 g Soda hinzu und versetzt unter Rühren tropfenweise mit 11 ml Chlorameisensäure-n-octylester. Man rührt noch vier Stunden bei Raumtemperatur und versetzt dann mit 200 ml Methanol und 1,5 g Holzkohle. Die Niederschläge werden durch Filtrieren entfernt und das Filtrat im Vakuum auf ein Volumen von ca. 100 ml eingedampft. Dann fügt man 100 ml Methylenchlorid hinzu, filtriert vom Niederschlag ab und dampft das Filtrat im Vakuum zum Sirup ein. Man erhält so die Titelverbindung als braunes Rohprodukt.

4.2 Extraktive Reinigung und Abspaltung der Schutzgruppen, Isolierung von Reinisomicin

Der Sirup wird in 50 ml der Unterphase des Zweiphasensystems n-Butanol/Ammoniak (konz.)/n-Hexan (Verhältnis 4,8/5,0/0,2 — jeweils Volumenteile) gelöst und die Lösung mit 50 ml der Oberphase des gleichen Systems gut durchgerührt. Die Oberphase wird im Vakuum eingedampft und der so erhaltene Sirup in 70 ml Wasser mit 40 g Bariumhydroxidhydrat 7 Stunden unter Rückfluss erhitzt. Dann wird in der Hitze mit 20% $H_2SO_4$ auf pH 5 gestellt, vom Bariumsulfat abzentrifugiert, das Zentrifugat durch Ausrühren mit Anionenaustauscherharz (Lewatit MP 500, OH⊖-Form) auf pH 10 bis 11 gestellt, vom Harz abfiltriert und das Filtrat (ca. 400 ml) über eine Kationenaustauschersäule (2,5 × 20 cm, Lewatit CNP-LF, $NH_4^{\oplus}$-Form, 100 ml) gegeben. Man wäscht mit 1,3 l Wasser nach und eluiert das Sisomicin anschliessend mit 200 ml 5%igem wässrigem Ammoniak. Nach Gefriertrocknen isoliert man 6,0 g = 85% farblosen Feststoff, Reinheitsgrad ca. 95%.
$[\alpha]_D^{22} = +188°$ (C = 1,0 $H_2O$).

Beispiel 5
Penta-N-methyloxycarbonylsisomicin, Rohrprodukt

9 g 50%iges Rohsisomicin in 100 ml Methanol werden unter Eiskühlung mit 3,5 g Dimethylpyrocarbonat versetzt.

Nach einer halben Stunde wird im Vakuum vom Lösungsmittel befreit und getrocknet.
Ausbeute: 13,8 g Rohprodukt;
(Rf der Titelverbindung als Hauptprodukt 0,33, Laufmittelsystem: Chloroform mit 10 Vol.-% Methanol).

Beispiel 6
6.1 Reinsisomicin aus Rohsisomicin über 1,2',-3,6'-Tetra-N-methyloxycarbonylsisomicin

170 ml Rohsisomicinlösung mit einem Sisomicingehalt von 20,3 g Sisomicin werden mit 375 ml Wasser und 100 ml Methanol verdünnt und bei einer Innentemperatur von −8 bis −10°C innerhalb einer Stunde mit einer Lösung von 28 ml Dimethylpyrocarbonat in 60 ml Methanol unter Rühren versetzt. Nach vollständiger Reaktion (DC-Kontrolle im Laufmittelsystem G) wird im Vakuum eingedampft und bis auf einen amorphen Feststoff getrocknet. Die Titelverbindung besitzt als Hauptkomponente einen Rf-Wert von 0,48 (Laufmittelsystem G).

6.2 Extraktive Reinigung und Isolierung von 1,2',3,6'-Tetra-N-methyloxycarbonylsisomicin

Der Rückstand aus Beispiel 6.1 wird in 340 ml konzentriertem wässrigem Ammoniak gelöst. Diese Lösung extrahiert man 20mal mit je 450 ml eines Gemisches aus 340 ml Methylenchlorid, das mit konzentriertem wässrigem Ammoniak gesättigt wurde und 135 ml n-Hexan, wobei das Tetra-N-methyloxycarbonylsisomicin in die organische Phase übertritt. Die vereinigten Extraktphasen werden im Vakuum vom Lösungsmittel befreit und der so erhaltene Rückstand in 200 ml konzentriertem wässrigem Ammoniak gelöst und 20mal mit je 265 ml eines Gemisches aus einem Volumenteil n-Hexan und 2,5 Volumenteilen Methylenchlorid, das mit konzentriertem wässrigem Ammoniak gesättigt wurde, extrahiert. Die vereinigten Extrakte werden im Vakuum vom Lösungsmittel befreit und bis zu einem amorphen Feststoff getrocknet.
Ausbeute: 28,0 g;
Rf = 0,48 (Laufmittelsystem G);
$[\alpha]_D^{20} = +153°$ (c = 1,0 Methanol).

6.3 Abspaltung der Schutzgruppen, Isolierung von Reinsisomicinsulfat

28 g 1,2',3,6'-Tetra-N-methyloxycarbonyl-sisomicin aus Beispiel 6.2 werden in 560 ml Wasser aufgenommen. Man versetzt mit 84 g Bariumhydroxidoctahydrat und erhitzt anschliessend in einem Druckreaktionsgefäss für ca. 20 Minuten auf 190°C, bei 30 bar Druck unter Stickstoffatmosphäre. Nach dem Abkühlen werden die Bariumsalze durch Zugabe von festem Kohlendioxid gefällt; man saugt von den ausgefallenen Salzen ab, wäscht den Rückstand mit Wasser gut aus und entionisiert die Filtrate durch Rühren mit 400 ml basischem Ionenaustauscherharz (Lewatit MP 500, OH⊖-Form). Nach Abtrennen des Harzes wird die so erhaltene wässrige Lösung von Sisomicinbase über eine Säule mit 370 ml Kationenaustauscherharz (Lewatit CNP, $NH_4^{\oplus}$-Form) gegeben, wobei das Sisomicin an das Harz gebunden wird. Man wäscht mit 1000 ml Wasser und eluiert anschliessend das

Sisomicin mit 5%igem wässrigem Ammoniumhydroxid. Nach Abdampfen des Ammoniaks verbleibt Sisomicin als farbloser Feststoff. Dieser wird in 140 ml Wasser gelöst und die alkalische Lösung mit verdünnter Schwefelsäure auf pH 4,8 gestellt. Man rührt mit Aktivkohle, filtriert und lyophilisiert das Filtrat.
Ausbeute an Sisomicinsulfat 27,3 g.
$[\alpha]_D^{20} = +106°$ (c = 1,0 $H_2O$).

Beispiel 7
Entblockierung von 1,2',3,6'-Tetra-N-methyloxycarbonylsisomicin zu Sisomicin

20 g 1,2',3,6'-Tetra-N-methyloxycarbonyl-sisomicin werden in 180 ml Dimethylsulfoxid und 60 ml Wasser mit 40 g Kaliumhydroxid für 50 Stunden bei 35°C gerührt. Zu diesem Zeitpunkt ist die Reaktion gemäss DC vollständig (Laufmittelsystem E).

Man versetzt mit 333 ml $H_2O$ und 77 ml konzentriertem Ammoniak und gibt diese Lösung über eine Säule, die 230 ml Kationenaustauscher (Lewatit CNP, $NH_4^{\oplus}$-Form) enthält. Man eluiert mit 200 ml 3%igem Ammoniak und vereinigt das Eluat mit der vor dem Eluieren erhaltenen ausfliessenden Lösung. Das Ammoniak wird im Vakuum verdampft, die zurückbleibende Lösung mit 500 ml Wasser verdünnt und auf eine Säule mit 170 ml Kationenaustauscher (Lewatit CNP, $NH_4^{\oplus}$-Form) gegeben. Der Austauscher wird gut mit Wasser gewaschen. Man eluiert mit 5%igem Ammoniak, vereinigt die Sisomicin enthaltenden Fraktionen und dampft im Vakuum zur Trockene ein.

Der Rückstand wird in Wasser aufgenommen und lyophilisiert. Man erhält so Sisomicinbase als farblosen Feststoff.
$[\alpha]_D^{22} = +189°$ (c = 1,0 $H_2O$).

Beispiel 8
1,2',3,6'-Tetra-N-ethyloxycarbonyl-sisomicin aus Rohsisomicin

100 ml Rohsisomicinlösung mit einem Sisomicingehalt von 22 g werden mit 700 ml Wasser und 500 ml Ethanol verdünnt. Bei −10°C tropft man langsam 39 ml Diethylpyrocarbonat in 78 ml Methanol zu. Nach quantitativer Reaktion (DC-Kontrolle; Laufmittelsystem G) wird im Vakuum das Lösungsmittel abgedampft. Der Rückstand wird in 300 ml konzentriertem Ammoniumhydroxid gelöst und diese Lösung 8mal mit je 660 ml eines Extraktionsmittels, bestehend aus 10 Volumenteilen Essigsäureethylester und 1 Volumenteil n-Hexan (die Mischung mit konzentriertem Ammoniumhydroxid gesättigt) ausgeschüttelt. Die vereinigten Extrakte werden im Vakuum eingedampft und getrocknet.
Ausbeute 35 g.
$^{13}$C-NMR ($CD_3OD/CDCl_3$):
δ = 50,86 (c-1), 49,91 (c-2); 46,33 (c-2'); 42,87 (c-6'); 157,94, 157,73, 157,29 und 157,22 (>c-o) ppm.

Das so erhaltene Produkt kann wie in den Beispielen 6.3 oder 7 beschrieben entblockiert werden.

Beispiel 9
Reinigung von Rohgentamicin

350 ml einer Rohgentamicin-C-Lösung mit einem Gentamicin-C-Gehalt von etwa 32 g (Anteile der Gentamicin-C-Komponenten in Gew.-%:

Gentamicin $C_{1a}$ = 25%
Gentamicin $C_1$ = 34%
Gentamicin $C_2$ = 41%)

werden bei 5 bis 10°C langsam unter Rühren mit 55 ml Acetanhydrid versetzt. Nach erfolgter Zugabe wird im Vakuum vom Lösungsmittel befreit, der Rückstand mit Toluol versetzt und erneut im Vakuum eingedampft. Das so erhaltene Rohgemisch wird mit 75 ml Wasser gelöst. Diese Lösung entionisiert man durch Rühren mit Anionenaustauscherharz (Lewatit MP 500, $OH^{\ominus}$-Form). Die nach Abtrennen des Austauscherharzes erhaltene Lösung wird im Vakuum eingedampft. Den Rückstand löst man in 160 ml Ethanol und 30 ml Wasser, versetzt mit 45 g Natriumcarbonat und tropft unter Rühren bei 50°C eine Lösung von 45 ml Chlorameisensäure-n-butylester in 45 ml Aceton innerhalb von etwa 1,5 Stunden zu. Man lässt abkühlen, versetzt mit 200 ml Toluol und dampft im Vakuum die Lösungsmittel ab. Der Rückstand wird in 300 ml Ethanol aufgenommen und gut durchgerührt. Dann filtriert man vom Ungelösten ab, wäscht mit Ethanol nach und dampft die vereinigten Filtrate im Vakuum ein. Man erhält so ein Rohgemisch, welches als Hauptkomponenten 1,2',3,-6'-Tetra-N-acetyl-3''-N-(n-butyloxycarbonyl)-gentamicin $C_{1a}$, 1,2',3,6'-Tetra-N-acetyl-3''-N-(n-butyloxycarbonyl)-gentamicin $C_2$ sowie 1,2',3-Tri-N--acetyl-3'',6'-di-N-(n-butyloxycarbonyl)-gentamicin $C_1$ enthält. Zur Abtrennung von den Verunreinigungen wird der Rückstand in 500 ml konzentriertem Ammoniumhydroxid gelöst. Man extrahiert diese Lösung mit 150 ml-Portionen der Unterphase eines Systems aus 7 Volumenteilen Methylenchlorid, 1,0 Volumenteilen Propanol-2 und 1 Volumenteil konzentriertem Ammoniumhydroxid. Die Gentamicin-C-derivate werden so in das Extraktionsmittel überführt, die Beiprodukte verbleiben in der Ammoniumhydroxidphase. Zur weiteren Reinigung werden die vereinigten Extraktphasen im Vakuum eingedampft und der dabei anfallende Rückstand wie oben beschrieben nochmals der Extraktion unterworfen. Der dabei nach Eindampfen der Extraktphase anfallende Sirup besteht aus einem Gemisch von 1,2',3,6'-Tetra-N-acetyl-3''-N-(n-butyloxycarbonyl)--gentamicin $C_{1a}$, 1,2',3,6'-Tetra-N-acetyl-3''-N-(n--butyloxycarbonyl)-gentamicin $C_2$ und 1,2',3-Tri--N-acetyl-3'',6'-di-N-(n-butyloxycarbonyl)-gentamicin $C_1$. Zur Abspaltung der Schutzgruppen löst man 30 g dieser Verbindung in 150 ml heissem Wasser und erhitzt nach Zusatz von 80 g Bariumhydroxid Octahydrat für 8 Stunden am

Rückfluss (Heizbadtemperatur ca. 150°C). Nach Abkühlen fällt man die Bariumsalze durch Zugabe von festem Kohlendioxid und filtriert den Niederschlag ab. Das Filtrat wird mit basichem Ionenaustauscherharz (Lewatit, MP 500, OH⊖-Form) entionisiert. Aus der Lösung wird die Gentamicin-C-Base durch Binden an Kationenaustauscherharz (Lewatit, CNP-LF, $NH_4^{\oplus}$-Form) entfernt. Das so erhaltene Harzaddukt wird mit Wasser gewaschen. Dann wird die Gentamicin-C-Base mit 5%igem wässrigem Ammoniumhydroxid eluiert. Das Eluat wird im Vakuum eingedampft und der Rückstand gefriergetrocknet.

Man erhält so Gentamicin-C-Base als farblosen Feststoff, der in das Sulfatsalz überführt wird, indem man die wässrige Lösung mit Schwefelsäure behandelt und dann lyophilisiert. $[\alpha]_D^{20}$ = +101° (c = 1,06 $H_2O$) als Sulfatsalz.

Beispiel 10

Abtrennung von Gentamicin $C_1$ aus dem Gentamicin-C-Komplex

70 ml einer Rohgentamicin-C-Lösung mit einem Gentamicin-C-Gehalt von etwa 6,5 g (Anteile der Gentamicin-C-Komponenten in Gew.-%:

Gentamicin $C_{1a}$ = 25%
Gentamicin $C_1$ = 34%
Gentamicin $C_2$ = 41%)

werden bei 5 bis 10°C langsam unter Rühren mit 11 ml Acetanhydrid versetzt. Nach erfolgter Zugabe wird im Vakuum vom Lösungsmittel befreit, der Rückstand mit Toluol versetzt und erneut im Vakuum eingedampft. Das so erhaltene Rohgemisch wird mit 75 ml Wasser gelöst und diese Lösung entionisiert man durch Rühren mit Anionenaustauscherharz (Lewatit, MP 500, OH⊖-Form).

Die nach Abtrennen des Austauscherharzes erhaltene Lösung wird im Vakuum eingedampft. Den Rückstand löst man in 42 ml Ethanol und 8 ml Wasser, versetzt mit 9,25 g Natriumcarbonat und tropft unter Rühren bei 50°C eine Lösung von 9,3 ml Chlorameisensäure-n-butylester in 9 ml Aceton innerhalb von etwa 0,5 Stunden zu. Man lässt abkühlen, versetzt mit 100 ml Toluol und dampft im Vakuum die Lösungsmittel ab. Der Rückstand wird in 100 ml Ethanol aufgenommen und gut durchgerührt. Dann filtriert man vom Ungelösten ab, wäscht mit Ethanol nach und dampft die vereinigten Filtrate im Vakuum ein. Man erhält so ein Rohgemisch, welches als Hauptkomponenten 1,2',3,6'-Tetra-N-acetyl-3"-N-(n-butyloxycarbonyl)-gentamicin $C_{1a}$, 1,2',3,6'-Tetra-N-acetyl-3"-N-(n-butyloxycarbonyl)-gentamicin $C_2$ sowie 1,2',3-Tri-N-acetyl-3",6'-di-N-(n-butyloxycarbonyl)-gentamicin $C_1$ enthält.

Zur Abtrennung des Gentamicin-$C_1$-Derivates wird der Rückstand in 100 ml konzentriertem Ammoniumhydroxid gelöst. Man extrahiert diese Lösung mit 150 ml der Unterphase eines Systems aus 7 Volumenteilen Methylenchlorid, 0,4 Volumenteilen Propanol-2 und 1 Volumenteil konzentriertem Ammoniumhydroxid. Das Gentamicin-$C_1$-Derivat wird so in das Extraktionsmittel überführt, die $C_{1a}$- und $C_2$-Derivate verbleiben in der Ammoniumhydroxidphase. Zur weiteren Reinigung wird die Methylenchloridphase noch zweimal mit je 100 ml konzentriertem Ammoniumhydroxid ausgeschüttelt. Anschliessend dampft man sie im Vakuum ein und trocknet bis zur Gewichtskonstanz. Man erhält so reines 1,2',3-Tri-N-acetyl-3",6'-di-N-(n-butyloxycarbonyl)-gentamicin $C_1$ als farblosen Feststoff.

$^{13}$C-NMR ($CD_3OD$) Rotamerengemisch aus zwei Rotameren:
δ = 160,342, 159,909, 158,917 und 158,545 (Urethan c=o); 101,672 und 101,241 (c-1'); 99,816 und 98,384 (c-1"); 172-174 (Acetyl c=o); 14,025 (Urethan $-CH_3$); 20 (Acetyl $-CH_3$) ppm.

Zur Abspaltung der Schutzgruppen löst man 3 g dieser Verbindung in 15 ml heissem Wasser und erhitzt nach Zusatz von 8 g Bariumhydroxid Octahydrat für 8 Stunden am Rückfluss (Heizbadtemperatur ca. 150°C). Nach Abkühlen fällt man die Bariumsalze durch Zugabe von festem Kohlendioxid und filtriert den Niederschlag ab. Das Filtrat wird mit basischem Ionenaustauscherharz (Lewatit, MP 500, OH⊖-Form) entionisiert. Aus der Lösung wird die Gentamicin-$C_1$-Base durch Binden an Kationenaustauscherharz (Lewatit, CNP-LF, $NH_4^{\oplus}$-Form) entfernt. Das so erhaltene Harzaddukt wird mit Wasser gewaschen. Dann wird die Gentamicin-$C_1$-Base mit 5%igem wässrigem Ammoniumhydroxid eluiert. Das Eluat wird im Vakuum eingedampft und der Rückstand gefriergetrocknet.

Man erhält so Gentamicin-$C_1$-Base als farblosen Feststoff.

Beispiel 11

Herstellung von 3"-N-Desmethyl-3"-N-ethylsisomicin aus Rohsisomicin

11a) 1,2',3,6'-Tetra-N-acetyl-3"-N-ethyl-sisomicin (Rohprodukt)

1,04 l Rohsisomicinlösung mit einem Sisomicingehalt von ca. 120 g werden auf ca. 5°C gekühlt. Bei dieser Temperatur tropft man 210 ml Acetanhydrid innerhalb von 45 Minuten unter gutem Rühren und Kühlen (Aceton/Trockeneis, zu Beginn stärker exotherme Reaktion) zu dieser Lösung.

Man stellt mit 50%iger Natronlauge auf pH 4,5, lässt dann auf Raumtemperatur kommen und verdünnt mit 430 ml Ethanol. Zu dieser Lösung gibt man bei 0° 82 ml Acetaldehyd und rührt etwa 30 Minuten bei Raumtemperatur.

Dann tropft man innerhalb 180 Minuten und unter gutem Rühren eine Lösung von 17 g Natriumboranat in 45 ml 10%iger Natronlauge und 920 ml Propanol-2 und 104 ml Wasser dazu. Nach 30 Minuten Nachrühren ist die Umsetzung gemäss DC quantitativ (Laufmittelsystem G). Man dampft nach Neutralisieren mit NaOH auf

ca. 600 ml im Vakuum ein. Das so erhaltene Konzentrat enthält als Hauptprodukt 1,2',3,6'-Tetra-N-acetyl-3"-N-ethylsisomicin.
Rf = 0,50 Laufmittelsystem G.

11b) 1,2',3,6'-Tetra-N-acetyl-3"-N-ethylsisomicin-Reinprodukt durch Extraktion

Aus dem nach Beispiel 11a) erhaltenen Konzentrat wird die Ethylverbindung durch Extraktion isoliert. Man versetzt das Konzentrat mit 1,4 l konzentriertem Ammoniumhydroxid und extrahiert in einem 6 l Scheidetrichter durch Rühren mit einem Flügelrührer (750 U/min) zunächst dreimal mit je 180 ml der Unterphase eines Gemisches aus Methylenchlorid-Propanol-1-$NH_4OH_c$ im Verhältnis 7:0,5:1 (jeweils Gewichtsteile), wobei die lipophilen Verunreinigungen abgetrennt werden. Die Unterphasen werden jeweils verworfen, die Wasserphase enthält die Titelverbindung. Zur Extraktion des gewünschten Produktes aus dieser Wasserphase rührt man sie 15mal mit je 700 ml der Unterphase des Gemisches Methylenchlorid-Propanol-1-$NH_4OH_c$ — im Verhältnis 7:1, 5:1 (Vol.-Teile) aus — indem man wie oben beschrieben verfährt. Insgesamt werden so ca. 160 g der Titelverbindung = 92% bezogen auf eingesetztes Sisomicin, die sich nun in den Unterphasen befinden, erhalten.
Reinheit ca. 95%.
$[\alpha]_D^{22} = +168°$ (c = 1,0 $CH_3OH$).

11c) Oxidative Dealkylierung zu 1,3,2',6'-Tetra-N-acetyl-3"-N-desmethyl-3"-N-ethylsisomicin und 1,3,2',6'-Tetra-N-acetylsisomicin

17 g der wie bei 11b) beschriebenen dargestellten 3"-N-Ethylverbindung werden in 225 ml Wasser gelöst. Man versetzt unter Eiskühlung mit 11 g Natriumhydroxid. Dann fügt man 85 ml Ethanol zu und kühlt auf ca. −20°C. Bei dieser Temperatur gibt man unter Rühren 30 g Kaliumhexacyanoferrat-(III) innerhalb 60 Minuten in 10 Portionen dazu. Nach Zugabe der letzten Probe wird noch 30 Minuten nachgerührt — gemäss DC (Laufmittelsystem G) ist die Umsetzung dann quantitativ.

Zur Aufarbeitung wird unter Rühren und Kühlen mit 30%iger Schwefelsäure auf pH 7 gestellt und dann mit einer Lösung von 30 g Kupfersulfat in 55 ml Wasser zur Fällung von Kupferhexacyanoferrat versetzt. Man lässt unter Rühren auf Raumtemperatur kommen, entfernt die Niederschläge durch Zentrifugieren, wäscht 4mal mit Wasser aus, stellt die vereinigten Zentrifugate mit 50%iger Natronlauge auf pH 7 und dampft im Vakuum bis zum dünnen Sirup ein (Temperatur ca. 50 bis 60°C). Man nimmt in 40 ml Methanol auf und versetzt unter gutem Rühren nacheinander mit 80 ml Aceton und 40 ml Essigester. Von den dadurch ausgefällten anorganischen Salzen wird abgesaugt, der Rückstand wird gut mit Aceton/Methanol/Essigester (Verhältnis = 2:1:1) ausgewaschen und die vereinigten Filtrate mit 40 ml neutral gewaschenem Ionenaustauscherharz Lewatit SC 108 ($H^{\oplus}$-Form) durch kurzes Rühren kupferfrei gemacht. Die Lösung wird dadurch zunehmend saurer — bei pH 3 saugt man vom Harz ab, wäscht mit Methanol nach und neutralisiert das Filtrat möglichst rasch mit festem Natriumcarbonat. Die so erhaltene Lösung wird im Vakuum zur Trockene eingedampft (DC-Kontrolle im System G). Man erhält so ein Produktgemisch bestehend aus 1,2',3,6'-Tetra-N-acetyl-3"-N-desmethyl-3"-N-ethylsisomicin als Hauptkomponente und 1,2',3,6'-Tetra-N-acetyl-sisomicin als Nebenprodukt.

11d) Abtrennung der 3"-N-Desmethyl-3"-N-ethylverbindung als 3"-N-(n-Octyloxycarbonyl)-derivat

Zur selektiven N-Carbomethoxylierung von 1,2',3,6'-Tetra-N-acetylsisomicin werden 10 g des wie oben dargestellten Oxidationsgemisches in 50 ml Methanol und 50 ml Aceton gelöst.

Man stellt die Lösung mit gesättigter methanolischer Natronlauge auf pH 11. Nun tropft man unter gutem Rühren innerhalb von 5 Minuten 0,2 ml Chlorameisensäuremethylester in 0,4 ml Aceton zu, man rührt noch 30 Minuten nach, stellt wie oben auf pH 11 und wiederholt die Zugabe des Chlorameisensäureesters und die pH-Regulierung wie beschrieben noch dreimal. 1,2',3,6'-Tetra-N-acetyl-sisomicin ist vollständig in 1,2',3,6'-Tetra-N-acetyl-3"-N-methyloxycarbonyl-sisomicin umgewandelt worden, wohingegen die 3"-N-Desmethyl-3"-N-ethylverbindung wegen der vergleichsweise geringeren Reaktivität der 3"-Ethylaminogruppe nicht reagiert hat. Im 1,2',3,6'-Tetra-N-acetyl-3"-N-methyloxycarbonyl-sisomicin sind alle Aminogruppen blockiert, 1,2',3,6'-Tetra-N-acetyl-3"-N-desmethyl-3"-N-ethyl-sisomicin enthält noch eine acylierbare 3"-Ethylaminogruppe, die nun zur Lipophilisierung dieses Derivates durch Umsetzung mit Chlorameisensäure-n-octylester urethanisiert wird.

Man lässt dazu die oben erhaltene Lösung auf Raumtemperatur kommen. Dann fügt man 10 g Natriumcarbonat zu und versetzt unter gutem Rühren mit 5 ml Chlorameisensäure-n-octylester. Nach ca. 60 Minuten werden weitere 5 ml Chlorameisensäureester zugesetzt und nach einer weiteren Stunde arbeitet man auf (übersch. Chlorameisensäureester mit $NH_4OH$ abfangen).

Zum Fällen der anorganischen Salze fügt man unter Rühren 150 ml Aceton zu und saugt über eine Fritte ab. Man wäscht mit Methanol/Aceton = ¼ nach und dampft die vereinigten Filtrate im Vakuum bei ca. 50 bis 60°C ein bis zum Sirup (DC im System G).

Der so erhaltene Sirup besteht als Hauptprodukt aus 1,2',3,6'-Tetra-N-acetyl-3"-N-desmethyl-3"-N-ethyl-3"-N-(n-octyloxycarbonyl)-sisomicin und enthält 1,2'3,6'-Tetra-N-acetyl-3"-N-methyloxycarbonylsisomicin als Nebenprodukt. Das auf die vorstehende beschriebene Weise stark lipophilisierte 3"-N-Desmethyl-Derivat wird nun extraktiv rein abgetrennt. Hierzu wird der Sirup

in 80 ml Wasser aufgenommen, und durch Rühren mit 40 ml n-Butanol und 48 ml n-Hexan (ca. 10 Minuten) extrahiert man das 3"-N-(n-octyloxycarbonyl)-derivat quantitativ in die Oberphase. Sie wird abgetrennt und zweimal mit je 20 ml Wasser ausgerührt. Die Oberphase wird im Vakuum zum Sirup eingedampft. Die vereinigten Unterphasen, in denen sich das 3"-N-Methyl-oxycarbonyl-sisomicin befindet, werden auf Sisomicin aufgearbeitet.

11e) 3"-N-Desmethyl-3"-N-ethylsisomicin aus 1,2',3,6'-Tetra-N-acetyl-3"-N-desmethyl-3"-N-ethyl-3"-(n-octyloxycarbonyl)-sisomicin

Der durch Eindampfen der wie in 11d) beschrieben erhaltenen Oberphase gewonnene Sirup wird in 40 ml Wasser aufgenommen und nach Zugabe von 24 g Bariumhydroxid 8 $H_2O$ für 5 bis 6 Stunden erhitzt (Badtemperatur 150 °C). Das freiwerdende Octanol wird dabei abdestilliert (Reaktionskontrolle mit DC im System E). Man lässt auf ca. 100°C abkühlen, fällt die Bariumionen durch Zugabe von 30%iger Schwefelsäure (tropfenweise) bis pH 5 und lässt auf Raumtemperatur kommen. Man entfernt das Bariumsulfat durch Zentrifugieren, schüttelt das Zentrifugat mit 50 ml Methylenchlorid oder Essigester aus und rührt die Wasserphase mit 1 g Aktivkohle. Man filtriert von der Kohle ab und rührt das Filtrat mit ca. 120 ml Ionenaustauscherharz Lewatit MP 500, OH⊖-Form bis pH 11, wodurch 3"-N-Desmethyl-3"-N-ethylsisomicin in die freie Base überführt wird. Der Ionenaustauscher wird abgesaugt und mit Wasser nachgewaschen. Die vereinigten Filtrate werden auf einer Säule an Kationenaustauscherharz (Lewatit CNP-LF, $NH_4^{\oplus}$-Form) adsorbiert. (Säulenmasse 1×20 cm, Durchflussgeschwindigkeit ca. 3 Tropfen/sec.). Das Harz wird mit 1 bis 1,5 l Wasser gewaschen. Anschliessend wird das Produkt mit 50%igem $NH_4OH$ eluiert (Durchflussgeschwindigkeit ca. 3 Tropfen/sec). Man vereinigt die Fraktionen, die 3"-N-Desmethyl-3"-N-ethyl-sisomicin enthalten (insgesamt ca. 70 ml) und dampft im Vakuum zur Trockene ein. Durch Aufnehmen in Methanol/Methylenchlorid und erneutes Eindampfen erhält man das Produkt als farblosen Feststoff.

$^{13}C$-NMR($D_2O$):

$\delta$ = 68,20 (c-2"); 63,08 (c-3"); 51,35 (c-1); 49,58 (c-3); 45,85 (c-6'); 41,65 ($-CH_2-CH_3$); 11,90 ($-CH_2-CH_3$) ppm.

Zum Überführen in das entsprechende Sulfat wird eine Lösung von 3"-N-Desmethyl-3"-N-ethyl-sisomicin in Wasser mit verdünnter Schwefelsäure auf pH 4,8 gestellt. Anschliessend wird gefriergetrocknet.

$[\alpha]_D^{20} = +107°$ (c = 1,0 Wasser).

Beispiel 12

1,2',3,6'-Tetra-N-acetyl-3"-N-ethyl-sisomicin aus Rohsisomicin

In einem 100 l Emaille-Rührwerkskessel werden 33 l Rohsisomicinlösung mit einem Sisomicingehalt von ca. 3 kg unter Rühren auf 0 bis 2°C abgekühlt. Zu dieser Lösung gibt man innerhalb von 2 Stunden 6 l Essigsäureanhydrid unter kräftigem Rühren, wobei die Temperatur zwischen 5 und 7°C gehalten wird. Man lässt 30 Minuten nachrühren und versetzt dann unter Rühren und Kühlen mit 10%iger Natronlauge bis pH 4,2. Dann fügt man 12 l Ethanol zu und kühlt die Lösung auf 5°C. Bei dieser Temperatur gibt man unter Rühren 2,5 l vorgekühlten Acetaldehyd zu und rührt unter ständigem Durchleiten von Stickstoff für 30 Minuten eine Lösung von 390 g Natriumborhydrid, 1,3 l 10%iger Natronlauge und 26 l Propanol-2 in 6 l Wasser unter Rühren zu. Man rührt noch etwa 1 Stunde nach und engt die Reaktionslösung im Vakuum auf ein Endvolumen von 15 l ein.

Man versetzt diese Lösung mit 35 l konzentriertem wässrigem Ammoniumhydroxid und erhält so 50 l einer Rohlösung mit etwa 4,3 kg 1,2',3,6'-Tetra-N-acetyl-3"-N-ethyl-sisomicin als Hauptprodukt. Diese Lösung wird für die Extraktion mit Wasser bis zu einem Gehalt von 5,2 Gew.-% 1,2',3,6'-Tetra-N-acetyl-3"-N-ethylsisomicin verdünnt.

Beispiel 13

Reinigung von 1,2',3,6'-Tetra-N-acetyl-3"-N-ethyl-sisomicin-Rohprodukt durch kontinuierliche Gegenstromextraktion

Im ersten Teil des Verfahrens werden lipophile Verunreinigungen abgetrennt. Hierzu werden in einem ARD-Extraktor mit Nennweite 72 mm pro Stunde 13,0 l einer gemäss Beispiel 12 hergestellten wässrig-ammoniakalischen Lösung mit 5,2 Gew.-% 1,2',3,6'-Tetra-N-acetyl-3"-N-ethylsisomicin (≙ 686 g/h) gegen stündlich 5,4 l eines Extraktionsmittels bestehend aus einem mit 25%igem wässrigem Ammoniumhydroxid abgesättigten Gemisch von 7 Vol.-Teilen Dichlormethan und 0,25 Vol.-Teilen Propanol-2 gefahren. Die Extraktionsmittelphase, welche die lipophilen Verunreinigungen enthält, wird in einer Verdampfereinheit im Vakuum eingedampft, und das nach Kondensation anfallende Lösungsmittelgemisch wird in einem Vorratsgefäss auf die ursprünglichen Mischungswerte eingestellt. Es wird dem Verfahren dann wieder als Extraktionsmittel zugeführt.

Die von den lipophilen Verunreinigungen gereinigte Raffinatphase wird kontinuierlich einer zweiten ARD-Extraktoreinheit mit Nennweite 150 mm zugeführt und gegen stündlich 90 l eines Lösungsmittels, bestehend aus einer mit konzentriertem wässrigem Ammoniumhydroxid abgesättigten Mischung von 7 Vol.-Teilen Dichlormethan und 2 Vol.-Teilen Propanol-2 gefahren.

Am oberen Kolonnenende wird die wässrig-ammoniakalische Raffinatphase abgenommen. Sie besitzt einen Restgehalt an 1,2',3,6'-Tetra-N-acetyl-3"-N-ethyl-sisomicin von ca. 0,1 Gew.-% und enthält vor allem die hydrophilen Verunreinigungen. Die unten an der Kolonne abgezogene Extraktphase, ca. 107 kg pro Stunde, enthält 0,615 Gew.-% 1,2',3,6'-Tetra-N-acetyl-3"-N-ethyl-sisomicin, entsprechend 658 g Produkt pro Stunde. Die Extraktphase wird im Vakuum aufkonzentriert. Das eingedampfte Lösungsmittel wird wiedergewonnen und wieder in das Verfahren eingesetzt. Die Gesamtausbeute bei diesem Extraktionsverfahren beträgt ca. 97% an 1,2',3,6'-Tetra-N-acetyl-3"-N-ethylsisomicin. Die Reinheit des Produktes ist 95%ig.

Beispiel 14

Gemisch von 1,2',3,6'-Tetra-N-acetyl-3"-N-ethyl-3"-N-desmethyl-3"-N-isopropyloxycarbonyl-sisomicin und 1,2',3,6'-Tetra-N-acetyl-3"-isopropyloxycarbonyl-sisomicin

In einem 4 l Dreihalskolben mit Rührer, Tropftrichter und Innenthermometer wird eine Lösung von 180 g des nach Beispiel 13 erhaltenen 1,2',3,6'-Tetra-N-acetyl-3"-N-ethylsisomicin in 270 ml Methanol und 250 ml Wasser gelöst und unter Kühlen mit einer Lösung von 64 g Natriumhydroxid in 400 ml Wasser versetzt. Dann kühlt man auf eine Innentemperatur von −15 bis −17°C und versetzt unter weiterem Rühren bei dieser Temperatur tropfenweise mit einer Lösung von 240 g Kaliumhexacyanoferrat-(III) in 600 ml Wasser innerhalb von 3 Stunden. Nach beendeter Zugabe wird noch 45 Minuten nachgerührt. Man lässt auf 0°C kommen und versetzt mit 5 g Natriumsulfit, lässt auf Raumtemperatur kommen und gibt nacheinander 500 ml Methanol und 240 g wasserfreies Natriumcarbonat zu. Unter Rühren wird auf 40°C erwärmt und dann gibt man innerhalb von 3 Stunden 700 ml Chlorameisensäureisopropylester in 300 ml Aceton zu. Nach 15stündigem Rühren bei 40°C wird die Lösung im Vakuum soweit eingedampft, dass das Methanol entfernt ist. Die dabei anfallende Lösung wird in 3 l Wasser gegossen und anschliessend versetzt man unter Rühren mit 1,4 l konzentriertem wässrigem Ammoniumhydroxid. Man filtriert und extrahiert mit je 5 l der Unterphase des Gemisches aus 7 Vol.-Teilen Methylenchlorid, 2,5 Vol.-Teilen Propanol-2 und 2 Vol.-Teilen 25%igem wässrigem Ammoniumhydroxid, wobei die Titelverbindungen in die organische Phase wandern, welche im Vakuum zum Sirup eingedampft wird. Salzartige Verbindungen und gegebenenfalls vorhandene polare Beiprodukte verbleiben in der Ammoniakphase.

Der durch Eindampfen des Extraktionsmittels anfallende Sirup besteht aus 1,2',3,6'-Tetra-N-acetyl-3"-N-desmethyl-3"-N-ethyl-3"-N-isopropyloxycarbonyl-sisomicin als Hauptprodukt (Rf= 0,51 Laufmittelsystem G) und 1,2',3,6'-Tetra-N-acetyl-3"-N-isopropyloxycarbonyl-sisomicin als Nebenkomponente (Rf = 0,41 System G).

Beispiel 15

1,2',3,6'-Tetra-N-acetyl-3"-N-desmethyl-3"-N-ethyl-3"-N-isopropyloxycarbonyl-sisomicin

Die Titelverbindung wird durch Extraktion aus dem wie in Beispiel 14 beschrieben hergestellten Gemisch mit 1,2',3,6'-Tetra-N-acetyl-3"-N-isopropyloxycarbonyl-sisomicin erhalten. Zu dieser Extraktion werden 200 g des gemäss Beispiel 14 hergestellten sirupösen Produktgemisches in 1,5 l 25%igem wässrigem Ammoniumhydroxid gelöst. Diese Lösung wird 12mal mit je 3 l der Unterphase eines Gemisches aus 7 Vol.-Teilen Methylenchlorid, 0,6 Vol.-Teilen Propanol-2 und einem Vol.-Teil konzentriertem Ammoniak extrahiert. Die vereinigten Extrakte werden im Vakuum eingedampft und der Rückstand nochmals dem vorstehend beschriebenen Extraktionsverfahren unterworfen. Die dabei anfallende Extraktphase wird im Vakuum eingedampft und der Rückstand bis zur Gewichtskonstanz getrocknet. Man erhält so die Titelverbindung als amorphen Feststoff in reiner Form.

$^{13}C$-NMR ($CD_3OD$):

101,257 (c-1"); 50,709 (c-1); 66,563 (c-3"); 173,629, 173,549, 173,452, 173,971 (Acetyl-C=O); 159,909 [$(CH_3)_2CH$-O-CO-)]; 15,18 (3"-NH-$CH_2$-$CH_3$) ppm.

Aus den vereinigten ammoniakalischen Raffinatphasen erhält man durch Eindampfen 1,2',3,6'-Tetra-N-acetyl-3"-N-isopropyloxycarbonyl-sisomicin.

$^{13}C$-NMR ($CD_3OD$):

101,064 (c-1"); 50,693 (c-1); 66,258 (c-3"); 173,613, 173,452, 172,971, 172,923 (Acetyl-C=O); 159,828 [$(CH_3)_2CH$-O-CO-); 34,662 (N-$CH_3$) ppm.

Die Abspaltung der Schutzgruppen kann wie in Beispiel 16 beschrieben erfolgen. Das dabei erhaltene Sisomicin kann dann wiederverwendet werden.

Beispiel 16

3"-N-Desmethyl-3"-N-ethylsisomicin

95 g des gemäss Beispiel 15 hergestellten 1,2',3,6'-Tetra-N-acetyl-3"-N-desmethylethyl-3"-N-isopropyloxycarbonyl-sisomicin werden in 500 ml Wasser mit 300 g Bariumhydroxidoctahydrat am Rückfluss erhitzt. Nach Abkühlen fällt man die Bariumionen durch Zugabe von festem Kohlendioxid, filtriert vom Bariumcarbonat ab, behandelt das Filtrat mit basischem Ionenaustauscherharz (Lewatit MP 500, $OH^{\ominus}$-Form) bis pH 11 und gibt diese Lösung über eine mit Kationenaustauscherharz beschickte Säule (Lewatit CNP-LF, $NH_4^{\oplus}$-Form). Das Harz, an welches das 3"-N-Desmethyl-3"-N-ethyl-sisomicin gebunden ist, wird mit Wasser gewaschen. Anschliessend eluiert man die Titelverbindung mit 5%igem wässrigem Ammoniumhydroxid und dampft das Eluat im Vakuum bis zum vollständigen Entfernen des Ammoniaks ein. Man gefriertrocknet und erhält die Titelverbindung als farblosen Feststoff.

$[\alpha]_D^{20} = +188°$ (c = 1,0 $H_2O$).

Beispiel 17
1,2',3,6'-Tetra-N-acetyl-3"-N-desmethyl-3"-N--ethyl-sisomicin durch Mangandioxidoxidation

10 g 1,2',3,6'-Tetra-N-acetyl-3"-N-ethylsisomicin werden in 200 ml Tetrahydrofuran und 25 ml Methanol gelöst und mit 40 g Mangandioxid unter gutem Rühren erhitzt. Nach vollständiger Reaktion (Kontrolle durch Dünnschichtchromatographie im System G) werden die Niederschläge abfiltriert, gewaschen und das Filtrat im Vakuum zur Trockene eingedampft. Der Rückstand wird in Wasser gelöst und mit Holzkohle gerührt. Man filtriert und dampf die Filtrate im Vakuum zur Trockene ein, wobei die Titelverbindung als amorpher Feststoff ausfällt.

Rf = 0,35 (System G).

Beispiel 18
1,2',3,6'-Tetra-N-acetyl-3"-N-desmethyl-3"-N--ethyl-sisomicin durch Mangandioxidoxidation in Wasser

1 g 1,2',3,6'-Tetra-N-acetyl-3"-N-ethyl-sisomicin werden in 20 ml Wasser gelöst und bei 0°C mit 8 g aktiviertem Mangandioxid gerührt. Nach vollständiger Reaktion (Kontrolle durch DC im System G) wird der Niederschlag abfiltriert, gewaschen und das Filtrat im Vakuum zur Trockne eingedampft. Der Rückstand wird in 5 ml Methanol und 20 ml Methylenchlorid gelöst, filtriert und eingedampft. Man erhält die Titelverbindung als farblosen Feststoff.

Rf = 0,35 (System G).

Beispiel 19
1,2',3,6'-Tetra-N-acetyl-3"-N-desmethyl-3"-N--ethyl-sisomicin durch Mangandioxidoxidation in Wasser/Essigsäure

In einem 50 l Rührwerkskessel werden 1,2 kg = 1,87 Mol 1,2',3,6'-Tetra-N-acetyl-3"-N-ethyl-sisomicin in 24 l Wasser gelöst. Unter Rühren stellt man mit 20%iger Essigsäure auf pH 6,5 und gibt dann 9,6 kg aktiviertes Mangandioxid zu. Der pH wird mit Natronlauge auf 6,0 eingestellt, und man rührt 15 Stunden weiter. Dann wird der Ansatz über Filterplatten abgesaugt, der Filterrückstand ($MnO_2$) mit 24 l Wasser gewaschen und das Filtrat im Dünnschichtverdampfer zu einem Sirup aufkonzentriert. Ausbeute = 86,4% (ermittelt durch Gehaltsbestimmung des Sirups über HPLC).

Die Abspaltung der Schutzgruppen wird dann wie in Beispiel 16 beschrieben durchgeführt.

Beispiel 20
Reinsisomicin aus Rohsisomicin über 1,2',3,3",-6'-penta-N-acetylsisomicin

20.1 1,2',3,3",6'-Penta-N-acetylsisomicin (Rohprodukt)

339 g wässrige Rohsisomicinlösung mit einem Sisomicingehalt von 36 g werden im Vakuum auf 87 g eingedampft. Man verdünnt mit 108 ml Aceton und tropft bei 20°C 84,5 ml Essigsäureanhydrid unter Rühren zu. Nach einer Reaktionszeit von ca. 2,5 Stunden wird mit 72 ml $H_2O$ verdünnt und mit 30%iger Natronlauge auf pH 6,5 gestellt. Dann dampft man das Gemisch im Vakuum bis auf 322 g ein. Man versetzt mit 393 g konzentriertem wässrigen Ammoniak und belässt diese Lösung für ca. 18 Stunden bei Raumtemperatur. Sie enthält die Titelverbindung als Hauptprodukt (Rf = 0,24 in Laufmittelsystem G).

20.2 Extraktive Reinigung und Abspaltung der Schutzgruppen; Isolierung von Reinsisomicin

Die nach 20.1 erhaltene Lösung wird fünfundzwanzigmal mit je 322 g der Unterphase des im Phasengleichgewicht befindlichen Systems aus Methylenchlorid (7 Vol.-Teile), Propanol-2 (4 Vol.-Teile) und konzentriertem wässrigen Ammoniak (4 Vol.-Teile) extrahiert. Die Extrakte werden vereinigt, das Extraktionsmittel im Vakuum verdampft, und man erhält so 1,2',3,3",6'-Penta-N--acetyl-sisomicin als farblosen Rückstand.

Zur Abspaltung der Schutzgruppen und Isolierung des Reinsisomicins verfährt man wie in Beispiel 3.3 beschrieben. Man gewinnt Sisomicinsulfat in reiner Form mit 80% Ausbeute. Reinheitsgrad: 96,7%.

Beispiel 21
Gentamicin aus Rohgentamicin über 1,2',3,3",6'--Penta-N-acetylgentamicin-C

21.1 1,2',3,3",6'-Penta-N-acetylgentamicin-C (Rohprodukt)

100 g wässrige Rohgentamicinlösung mit einem Gentamicin-C-Gehalt von 9 g (Anteile der Gentamicin-C-Komponenten entsprechen den Verhältnissen aus Beispiel 9) werden im Vakuum auf 22 g eingedampft. Man verdünnt mit 27 ml Aceton und tropft bei 20°C 23 ml Essigsäureanhydrid unter Rühren zu. Nach einer Reaktionszeit von ca. 2,5 Stunden wird mit 17,2 ml $H_2O$ verdünnt und mit 30%iger Natronlauge auf pH 6,5 gestellt. Dann dampft man das Gemisch im Vakuum bis auf 80 g ein. Man versetzt mit 100 g konzentriertem wässrigen Ammoniak und belässt diese Lösung für ca. 1,8 Stunden bei Raumtemperatur. Sie enthält die Titelverbindung als Hauptprodukt.

21.2 Extraktive Reinigung und Abspaltung der Schutzgruppen; Isolierung von Gentamicin-C-Reinprodukt

Die bei 21.1 erhaltene Lösung wird fünfundzwanzigmal mit je 322 g der Unterphase des im Phasengleichgewicht befindlichen Systems aus Methylenchlorid (7 Vol.-Teile), Propanol-2 (3 Vol.-Teile) und konzentriertem wässrigen Ammoniak (3 Vol.-Teile) extrahiert. Die Extrakte werden vereinigt, das Extraktionsmittel im Vakuum verdampft, und man erhält so 1,2',3,3",6'-Penta-N-acetylgentamicin-C als farblosen Rückstand.

Zur Abspaltung der Schutzgruppen und Isolierung des Reingentamicin-C verfährt man wie in Beispiel 9 beschrieben.

$[\alpha]_D^{20} = +102{,}5°$ (c = 2,0 in $H_2O$) als Sulfatsalz.

**Patentansprüche**

1. Verfahren zur Herstellung reiner Aminoglycosyd-Antibiotika, dadurch gekennzeichnet, dass man

a) vorgereinigte Verbindungen der allgemeinen Formel

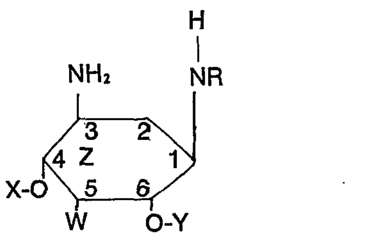

(I)

worin

X einen Rest der Formeln

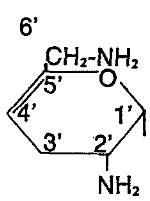

,

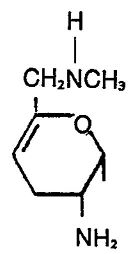

,

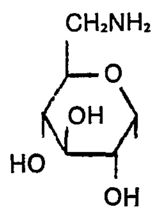

,

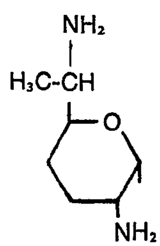

,

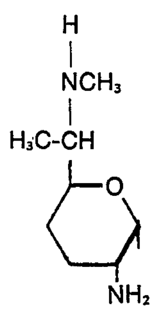

,

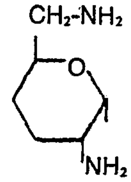

,

Y einen Rest der Formeln

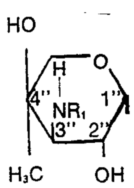

,

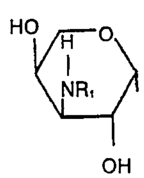

,

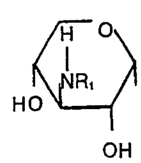

R Wasserstoff oder Ethyl,

$R_1$ eine $C_1$-$C_6$-Alkylgruppe und

ein Rest Z oder W Wasserstoff und der andere Rest Z oder W Wasserstoff oder Hydroxy bedeuten,

in an sich bekannter Weise in Verbindungen der allgemeinen Formel

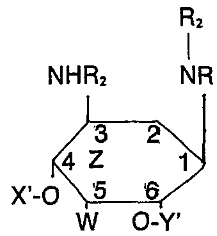

(II)

überführt, worin

X' für einen Rest der Formeln

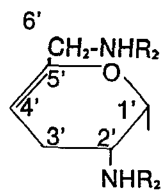

,

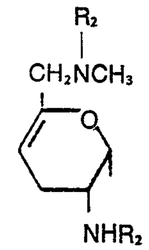

,

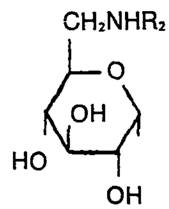

,

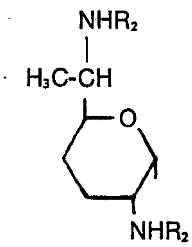

,

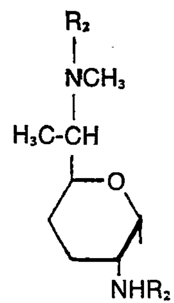

,

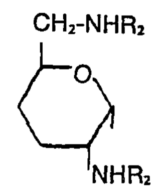

,

Y' für einen Rest der Formeln

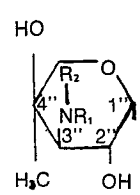

,

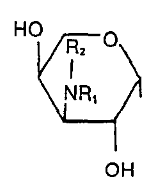

,

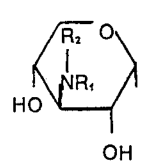

steht, die Reste $R_2$ gleich oder verschieden sind und Wasserstoff oder eine Acyl- oder Arylsulfenyl-Schutzgruppe darstellen, mit der Massgabe, dass maximal zwei der Reste $R_2$ für Wasserstoff stehen, und R, $R_1$, Z und W die oben angegebene Bedeutung haben, danach

b) gegebenenfalls die Verbindungen der Formel (II) an den nicht mit Schutzgruppen versehenen Aminogruppen chemisch abwandelt,

c) die gegebenenfalls derivatisierten Verbindungen der Formel (II) einer flüssig-flüssig-Extraktion in einem zweiphasigen wässrig/organischen Lösungsmittelsystem unterwirft und aus den Extrakten isoliert,

d) gegebenenfalls die erhaltenen gereinigten Verbindungen der Formel (II) an den nicht mit Schutzgruppen versehenen Aminogruppen chemisch abwandelt und

e) die Schutzgruppen in an sich bekannter Weise abspaltet.

2. Verfahren nach Anspruch 1, dadurch gekennzeichnet, dass man als Schutzgruppen $R_2$ Acylgruppen der Formeln

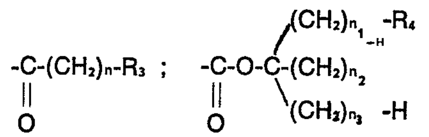

worin

$R_3$ und $R_4$ unabhängig voneinander Wasserstoff oder gegebenenfalls substituiertes Phenyl und

n, $n_1$, $n_2$ und $n_3$ unabhängig voneinander Zahlen von 0 bis 5 bedeuten,

oder Sulfenylschutzgruppen der Formel

$$-S—R_5$$

worin

$R_5$ gegebenenfalls substituiertes Phenyl, Di- oder Triphenylmethyl bedeutet,

einsetzt.

3. Verfahren nach Anspruch 1 oder 2, dadurch gekennzeichnet, dass die Verbindung der Formel (I) Sisomicin, 5-Episisomicin, Netilmicin, Gentamicin oder 3"-N-Desmethyl-3"-ethylsisomicin ist.

4. Verfahren nach Anspruch 2, dadurch gekennzeichnet, dass man

a) vorgereinigtes Sisomicin oder Gentamicin an den Aminogruppen per-acetyliert,

b) das so erhaltene Produkt einer flüssig-flüssig-Extraktion in einem zweiphasigen wässrig/organischen Lösungsmittelsystem unterwirft und aus den Extrakten das Penta-N-acetyl-sisomicin oder -gentamicin isoliert und

c) die Schutzgruppen in an sich bekannter Weise abspaltet.

5. Verfahren nach Anspruch 2, dadurch gekennzeichnet, dass man

a) vorgereinigtes Sisomicin an den Aminogruppen in 1,2',3 und 6'-Stellung acetyliert,

b) durch reduktive Alkylierung mit Acetaldehyd an der 3"-Aminogruppe ethyliert,

c) das so erhaltene Produkt einer flüssig-flüssig-Extraktion in einem zweiphasigen wässrig/organischen Lösungsmittelsystem unterwirft und aus den Extrakten das 1,2',3,6'-Tetra-N-acetyl-3"-N-ethylsisomicin isoliert,

d) das isolierte Produkt an der 3"-Aminogruppe in an sich bekannter Weise demethyliert und

e) die Schutzgruppen in an sich bekannter Weise abspaltet.

6. Verfahren nach Anspruch 1 bis 5, dadurch gekennzeichnet, dass man als wässrige Phase des Lösungsmittelsystems in Stufe c) wässriges Ammoniumhydroxid verwendet.

7. Verfahren nach Anspruch 6, dadurch gekennzeichnet, dass man solche Zweiphasensysteme einsetzt, die sich bilden, wenn man wässrige Ammoniumhydroxidlösungen mit einem organischen, nicht oder nur begrenzt mit wässrigem Ammoniumhydroxid mischbaren Lösungsmittel (A) und einem weiteren organischen Lösungsmittel (B) zusammenbringt, wobei (B) wahlweise mit wässrigem Ammoniumhydroxid mischbar sein kann, anschliessend bis zur Gleichgewichtseinstellung durchmischt, dann die Phasen voneinander trennt und für das betreffende Extraktionsverfahren einsetzt.

8. Verfahren nach Anspruch 7, dadurch gekennzeichnet, das man als organische Lösungsmittel (A) und (B) Methylenchlorid und Isopropylalkohol einsetzt.

9. Verfahren nach Anspruch 1 bis 8, dadurch gekennzeichnet, dass das Mengenverhältnis von organischer Phase zu wässriger Phase 0,5:1 bis 30:1 beträgt und die Extraktion bei Temperaturen zwischen 10 und 60°C durchgeführt wird.

10. Verfahren nach Anspruch 1 bis 9, dadurch gekennzeichnet, dass die Extraktion als Gegenstromextraktion durchgeführt wird.

**Claims**

1. Process for the preparation of pure aminoglycoside antibiotics, characterised in that

a) pre-purifiied compounds of the general formula

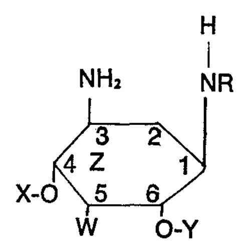

(I)

wherein

X denotes a radical of the formulae

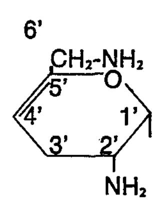

,

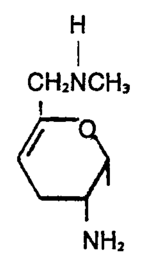

,

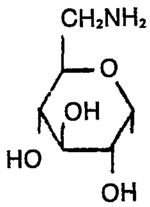,
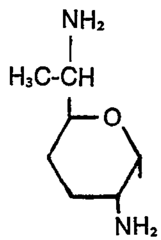,
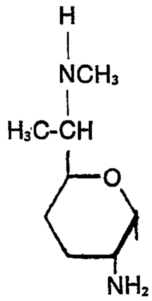,
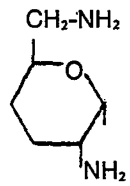,

Y denotes a radical of the formulae

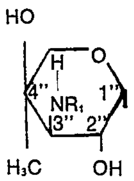,
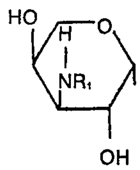,
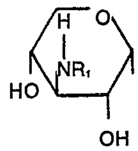

R denotes hydrogen or ethyl,

$R_1$ denotes a $C_1$-$C_6$-alkyl group, and

one of the radicals Z or W denotes hydrogen and the other radical Z or W denotes hydrogen or hydroxyl,

are converted into compounds of the general formula

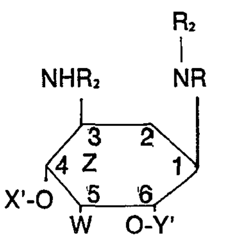
(II)

wherein

X' represents a radical of the formulae

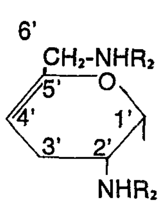,
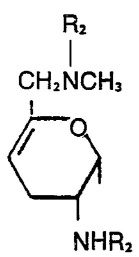,
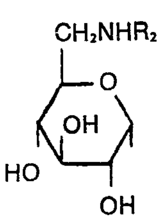,
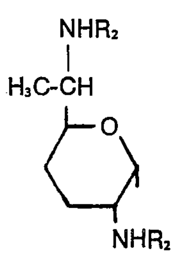,
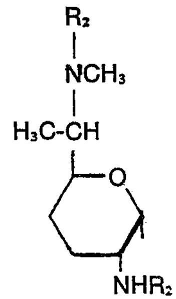,
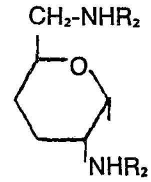,

Y' represents a radical of the formulae

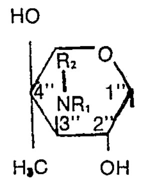,
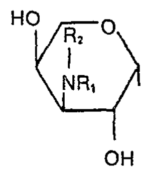,
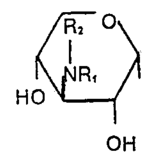

the radicals $R_2$ are identical or different and represent hydrogen or an acyl or arylsulphenyl protective group, with the proviso that at most two of the radicals $R_2$ represent hydrogen, and R, $R_1$, Z and W have the abovementioned meaning,

in a manner which is in itself known, and then

b) if appropriate, the compounds of the formula (II) are chemically modified on the amino groups which are not provided with protective groups,

c) the optionally derivatised compounds of the formula (II) are subjected to liquid/liquid extraction in a two-phase aqueous/organic solvent system and are isolated from the extracts, and

d) if appropriate, the resulting purified compounds of the formula (II) are chemically modified on the amino groups which are not provided with protective groups, and

e) the protective groups are spilt off in a manner which is in itself known.

2. Process according to Claim 1, characterised in that acyl groups of the formulae

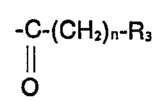 ; 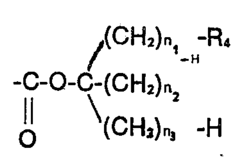

wherein

$R_3$ and $R_4$ independently of one another denote hydrogen or optionally substituted phenyl and

n, $n_1$, $n_2$ and $n_3$ independently of one another denote numbers from 0 to 5,

or sulphenyl protective groups of the formula

$$-S-R_5$$

wherein

$R_5$ denotes optionally substituted phenyl or di- or tri-phenylmethyl,

are employed as protective groups $R_2$.

3. Process according to Claim 1 or 2, characterised in that the compound of the formula (I) is sisomicin, 5-episisomicin, netilmicin, gentamicin or 3"-N-demethyl-3"-ethylsisomicin.

4. Process according to Claim 2, characterised in that

a) pre-purified sisomicin or gentamicin is peracetylated on the amino groups,

b) the product thus obtained is subjected to liquid/liquid extraction in a two-phase aqueous/organic solvent system and penta-N-acetyl-sisomicin or -gentamicin is isolated from the extracts and

c) the protective groups are split off in a manner which is in itself known.

5. Process according to Claim 2, characterised in that

a) pre-purified sisomicin is acetylated on the amino groups in the 1,2',3 und 6'-position,

b) the product is ethylated on the 3"-amino group by reductive alkylation with acetaldehyde,

c) the product thus obtained is subjected to liquid/liquid extraction in a two-phase aqueous/organic solvent system and 1,2',3,6'-tetra-N-acetyl-3"-N-ethylsisomicin is isolated from the extracts,

d) the isolated product is dementhylated on the 3"-amino group in a manner which is in itself known and

e) the protective groups are split off in a manner which is in itself known.

6. Process according to Claims 1 to 5, characterised in that aqueous ammonium hydroxide is used as the aqueous phase of the solvent system in stage c).

7. Process according to Claim 6, characterised in that the two-phase systems employed are those which are formed when aqueous ammonium hydroxide solutions are brought together with an organic solvent (A) which is immiscible with aqueous ammonium hydroxide or miscible with aqueous ammonium hydroxide only to a limited extent and a further organic solvent (B), it being possibles as desired, for (B) to be miscible with aqueous ammonium hydroxide, the components are then mixed thoroughly until equilibrium is established, and the phases are then separated from one another and used for the extraction process in question.

8. Process according to Claim 7, characterised in that methylene chloride and isopropyl alcohol are used as the organic solvents (A) and (B).

9. Process according to Claims 1 to 8, characterised in that the proportion of organic phase to aqueous phase is 0.5:1 to 30:1 and the extraction is carried out at temperatures between 10 and 60°C.

10. Process according to Claims 1 to 9, characterised in that the extraction is carried out as countercurrent extraction.

**Revendications**

1. Procédé de production d'antibiotiques du type aminoglycoside purs, caractérisé en ce que:

a) on transforme des composés préalablement purifiés, répondant à la formule générale:

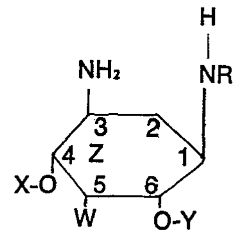

(I)

[dans laquelle

X est un radical répondant aux formules

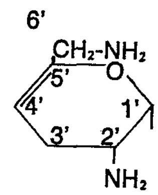
,
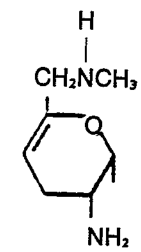
,
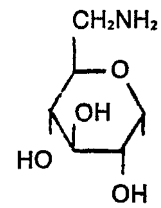
,
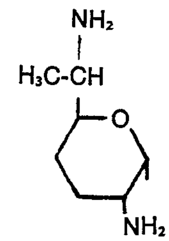
,
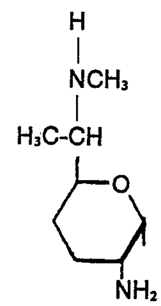
,
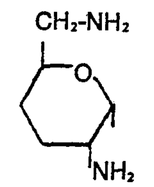
,

Y est un radical répondant aux formules

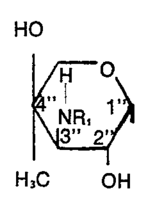
,
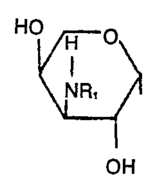
,
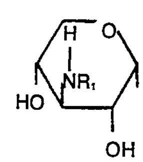

R est un atome d'hydrogène ou un groupe éthyle,

$R_1$ est un groupe alkyle en $C_1$-$C_6$ et

un radical Z ou W est un atome d'hydrogène et l'autre radical Z ou W est un atome d'hydrogène ou un groupe hydroxyle], de façon connue en soi en des composés de formule générale:

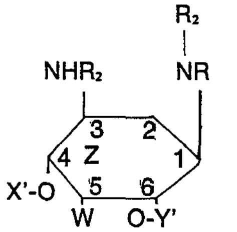

(II)

dans laquelle

X' est un radical répondant aux formules

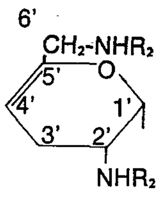
,
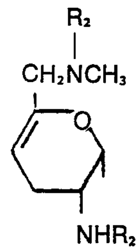
,
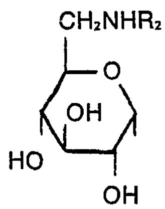
,
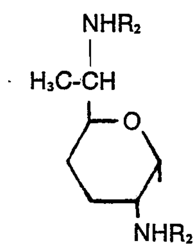
,
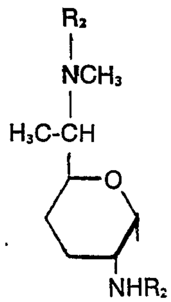
,
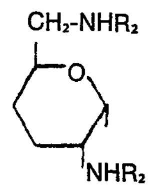
,

Y' est un radical répondant aux formules

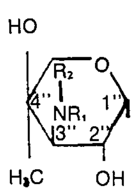
,
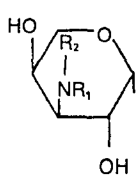
,
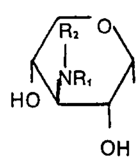

les radicaux $R_2$ sont identiques ou différents et représentent de l'hydrogène ou un groupe protecteur acyle ou arylsulfényle, étant bien entendu qu'au maximum deux des radicaux R représentent de l'hydrogène, et R, $R_1$, Z et W ont le sens indiqué ci-dessus; puis

b) éventuellement on transforme chimiquement les composés de formule (II) au niveau des groupes amino non pourvus de groupes protecteurs,

c) on soumet les composés de formule (II), éventuellement transformés en des dérivés, à une extraction liquide/liquide dans un système en deux phases solvants aqueux/organiques et on les isole des extraits,

d) éventuellement on transforme chimiquement les composés purifiés obtenus, de formule (II), au niveau des groupes amino non pourvus de groupes protecteurs, et

e) on scinde de façon connue en soi les groupes protecteurs.

2. Procédé selon la revendication 1, caractérisé en ce qu'on utilise comme groupes protecteurs $R_2$ des groupes acyle répondant aux formules:

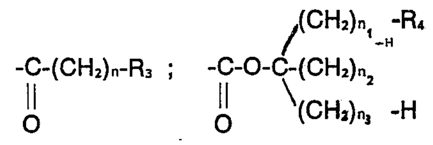

[dans lesquelles

$R_3$ et $R_4$ représentent, indépendamment l'un de l'autre, un atome d'hydrogène ou un groupe phényle éventuellement substitué, et

n, $n_1$, $n_2$ et $n_3$ représentent, indépendamment l'un de l'autre, des nombres valant 0 à 5],

ou des groupes protecteurs sulfényle de formule

$$-S—R_5$$

[dans laquelle $R_5$ est un groupe phényle éventuellement substitué, di- ou triphénylméthyle].

3. Procédé selon la revendication 1 ou 2, caractérisé en ce que le composé de formule (I) est la sisomicine, la 5-épisisomicine, la nétilmicine, la gentamicine ou la N-desméthyl-3''-éthyl-3''-sisomicine.

4. Procédé selon la revendication 2, caractérisé en ce que:

a) on peracétyle au niveau des groupes amino la sisomicine ou gentamicine préalablement purifiée,

b) on soumet le produit ainsi obtenu à une extraction liquide/liquide dans un système de solvants aqueux/organiques en deux phases et l'on isole des extraits la penta-N-acétyl-sisomicine ou -gentamicine, et

c) on scinde de façon connue en soi les groupes protecteurs.

5. Procédé selon la revendication 2, caractérisé en ce que:

a) on acétyle, au niveau des groupes amino en position 1,2',3 et 6', de la sisomicine préalablement purifiée,

b) par alkylation réductrice à l'aide d'acétaldéhyde, on éthyle au niveau du groupe amino en position 3'',

c) on soumet le produit ainsi obtenu à une extraction liquide/liquide dans un système de solvants aqueux/organique en deux phases et l'on isole des extraits la tétra-N-acétyl-1,2,3,6'-N-éthyl-3''-sisomicine,

d) on déméthyle de façon connue en soi, au niveau du groupe amino en position 3'', le produit isolé, et

e) on scinde de façon connue en soi des groupes protecteurs.

6. Procédé selon l'une des revendications 1 à 5, caractérisé en ce que l'on utilise comme phase aqueuse du système des solvants dans l'étape c) de l'hydroxyde d'ammonium aqueux.

7. Procédé selon la revendication 6, caractérisé en ce qu'on utilise des systèmes à deux phases qui se forment lorsque l'on combine des solutions aqueuses d'hydroxyde d'ammonium avec un solvant organique (A) non miscible avec de l'hydroxyde d'ammonium aqueux ou ne présentant qu'une miscibilité limitée avec cet hydroxyde d'ammonium, et avec un autre solvant organique (B), ce solvant (B) pouvant éventuellement être miscible avec de l'hydroxyde d'ammonium aqueux, puis l'on mélange jusqu'à obtention de l'équilibre, et l'on sépare ensuite les phases l'une de l'autre et on les utilise pour le procédé d'extraction en cause.

8. Procédé selon la revendication 7, caractérisé en ce qu'on utilise comme solvants organiques (A) et (B) le chlorure de méthylène et l'alcool isopropylique.

9. Procédé selon l'une des revendications 1 à 8, caractérisé en ce que le rapport des quantités de la phase organique à la phase aqueuse se situe entre 0,5:1 et 30:1 et en ce qu'on effectue l'extraction à des températures comprises entre 10 et 60°C.

10. Procédé selon l'une des revendications 1 à 9, caractérisé en ce qu'on effectue l'extraction sous forme d'une extraction à contre-courant.